(19) Europäisches Patentamt
European Patent Office
Office européen des brevets

(11) **EP 4 509 486 A1**

(12) **EUROPEAN PATENT APPLICATION**
published in accordance with Art. 153(4) EPC

(43) Date of publication:
**19.02.2025 Bulletin 2025/08**

(21) Application number: **23788357.4**

(22) Date of filing: **12.04.2023**

(51) International Patent Classification (IPC):
***C07B 33/00*** *(2006.01)* ***C11D 7/18*** *(2006.01)*
***C11D 7/54*** *(2006.01)* ***C11D 7/60*** *(2006.01)*
***C07D 213/61*** *(2006.01)* ***C07D 213/80*** *(2006.01)*
***C07D 213/82*** *(2006.01)* ***C09K 3/00*** *(2006.01)*

(52) Cooperative Patent Classification (CPC):
**C07B 33/00; C07D 213/61; C07D 213/80; C07D 213/82; C09K 3/00; C11D 3/39; C11D 3/395; C11D 7/00**

(86) International application number:
**PCT/JP2023/014846**

(87) International publication number:
**WO 2023/199939 (19.10.2023 Gazette 2023/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.04.2022 JP 2022066551**

(71) Applicant: **Kao Corporation**
**Tokyo 103-8210 (JP)**

(72) Inventors:
- **INAGAKI Kensuke**
  **Wakayama-shi, Wakayama 640-8580 (JP)**
- **Takano Ninna**
  **Nagoya-shi, Aichi 466-0828 (JP)**

(74) Representative: **Hoffmann Eitle**
**Patent- und Rechtsanwälte PartmbB**
**Arabellastraße 30**
**81925 München (DE)**

(54) **COMPOSITION**

(57) The present invention is a composition containing, (A) one or more compounds selected from a compound represented by the following general formula (A1) and a compound represented by the following general formula (A2) and water, the composition being acidic,

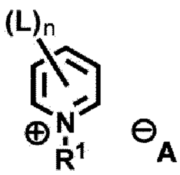

**(A1)**

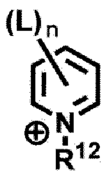

**(A2)**

wherein $R^1$ is an alkyl group with 1 or more and 24 or less carbons which may contain a heteroatom, $R^{12}$ is a group with 1 or more and 24 or less carbons in total which contains a carbon atom and a heteroatom of an ion paired with $N^+$ of the pyridine ring and which may contain a heteroatom different from the heteroatom, L is n halogen atoms, n being 1, 2 or 3, and L is bonded to any n different carbon atoms at positions 2, 4 and 6 of the pyridine ring, $A^-$ is an anion, and a group other than L may be bonded to a carbon atom other than the carbon atom bonded to L of the carbon atoms of the pyridine ring.

EP 4 509 486 A1

## Description

Field of the Invention

The present invention relates to a composition, a method for storing a compound, an oxidizing method, a bleaching method, use of a predetermined composition as an oxidizing agent and use of a predetermined composition as a bleaching agent.

Background of the Invention

An oxidizing agent composition is a composition containing a compound having an oxidizing effect on other substances (oxidizing agent). On a colored substance, this oxidizing effect can be recognized as a phenomenon such as decolorization, bleaching, discoloration or the like. Oxidizing agent compositions are used for various applications such as bleaching of hard articles in bathrooms, toilets, kitchens and others, mold removal, bleaching of textile products such as clothing and others, decomposition of substances causative of stains or odor, disinfection, bleaching of pulp, scouring of fibers and others.

Various stains forming in our living environment are dealt with by cleaning, bleaching, disinfection and others for the purpose of maintaining fine appearances, sanitizing and others. Especially, stains on places difficult to clean such as toilets, bathrooms, bath boilers, plumbing and others are difficult to remove only with cleaning agents composed mainly of surfactants or the like, and bleaching agents are often used for them. For example, dark stains on bath tubs in bathrooms, tile joints, door frames, window frames, controllers, corner sink strainers in kitchens and others are caused by pigments produced by mold such as fungi of the genus *Cladosporium* or the like, and difficult to remove only with cleansers or surfactants, and thus, removing methods through the bleaching action of aqueous sodium hypochlorite solutions are generally performed.

However, while mold removing agents using sodium hypochlorite are excellent in bleaching and cleaning performance, there are problems that they are highly irritating to eyes or the skin and have the distinctive chlorine odor, thus requiring care when used in small bathrooms or the like, and they generate the toxic gas if accidentally mixed with acidic cleaning agents.

Therefore, as application examples of oxidizing agent compositions not causing such problems, agents using oxygen-based bleaching agents have been proposed. For example, JP-A H3-220298 discloses a bleaching agent composition for hard surfaces using hydrogen peroxide, an alkali agent and a bleach activator in combination.

Further, JP-A 2009-155292 discloses a composition exhibiting high bactericidal and disinfection effects composed of a peroxide which produces hydrogen peroxide when dissolved in water, a chelating agent, a copper compound and a binder compound.

Further, JP-A 2002-80894 describes a bleaching agent composition used in such a manner that a highly viscous composition 1 containing hydrogen peroxide, an organic thickener, an organic chelating agent and water, and a highly viscous composition 2 containing an inorganic alkali, an organic thickener, a bleach activator and water are mixed when the composition is used.

Summary of the Invention

In oxygen-based bleaching agents, activated bleaching species (organic peroxy acids or the like) are produced by reacting, for example, hydrogen peroxide as an oxidizing agent with organic acid esters or the like as bleach activators, and objects are oxidized and bleached therewith. In the preparation of oxygen-based bleaching agents, it would be desirable in terms of handleability or the like if bleach activators could be stably supplied in aqueous solution form.

The present invention provides a composition which can be utilized as a bleach activator for oxygen-based bleaching agents or the like and which is stable in aqueous solution form.

The present invention relates to a composition containing, (A) one or more compounds selected from a compound represented by the following general formula (A1) and a compound represented by the following general formula (A2) [hereinafter referred to as component (A)] and water, the composition being acidic,

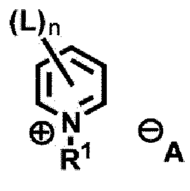

(A1)

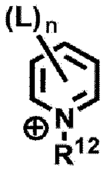

( A2 )

wherein $R^1$ is an alkyl group with 1 or more and 24 or less carbons which may contain a heteroatom, $R^{12}$ is a group with 1 or more and 24 or less carbons in total which contains a carbon atom and a heteroatom of an ion paired with $N^+$ of the pyridine ring and which may contain a heteroatom different from the heteroatom, L is n halogen atoms, n being 1, 2 or 3, and L is bonded to any n different carbon atoms at positions 2, 4 and 6 of the pyridine ring, $A^-$ is an anion, and a group other than L may be bonded to a carbon atom other than the carbon atom bonded to L of the carbon atoms of the pyridine ring.

Further, the present invention relates to a method for storing a compound including, storing component (A) in an acidic solution.

Further, the present invention relates to an oxidizing method including, bringing a treatment liquid prepared from the composition of the present invention into contact with an object in the presence of hydrogen peroxide.

Further, the present invention relates to a bleaching method including, bringing a treatment liquid prepared from the composition of the present invention into contact with an object in the presence of hydrogen peroxide.

Further, the present invention relates to use of the composition of the present invention for the production of oxidizing agents.

Further, the present invention relates to use of the composition of the present invention for the production of bleaching agents.

According to the present invention, provided is a composition which can be utilized as a bleach activator for oxygen-based bleaching agents or the like and which is stable in aqueous solution form.

It is known that organic acid esters have been conventionally used as bleach activators for hydrogen peroxide, an oxygen-based bleaching agent, as described above. In this combination, reactions for producing activated bleaching species proceed relatively efficiently in alkaline conditions, and proceed slowly in neutral conditions. Accordingly, bleaching agents or oxidizing agents using peroxy acids such as hydrogen peroxide and others tend to have reduced bleaching power or oxidizing power in neutral conditions. However, if the composition of the present invention is used in combination with hydrogen peroxide, oxidizing power or bleaching power due to hydrogen peroxide is not reduced even in neutral conditions, and excellent effects can be obtained.

## Embodiments of the Invention

### [Composition]

The reason why the composition of the present invention is excellent in stability is not wholly certain, but is inferred to be as follows. Under acidic conditions, active esters, common bleach activators, tend to be unstable as the active ester groups undergo protonation and become activated. On the other hand, component (A) of the present invention does not have an active ester group, and is not activated even under acidic conditions.

Further, the reason why excellent oxidizing power is exhibited in neutral conditions if the composition of the present invention is used in combination with hydrogen peroxide is not wholly certain, but it is inferred that this is because component (A) of the composition of the present invention has higher reactivity to hydrogen peroxide compared to general organic peroxy acid precursors, and thus, activated species are sufficiently formed even in neutral conditions. Note that it is inferred that reactivity of component (A) and hydrogen peroxide is extremely low under acidic conditions, and thus, component (A) can be stably present in the composition of the present invention even under the coexistence of hydrogen peroxide.

The mechanism of the present invention is not limited thereto.

Component (A) is one or more compounds selected from a compound represented by the above general formula (A1) and a compound represented by the above general formula (A2).

In the general formula (A1), $R^1$ is an alkyl group with 1 or more and 24 or less carbons which may contain a heteroatom. The alkyl group of $R^1$ has 1 or more, further 8 or more and further 12 or more, and 24 or less, further 20 or less and further 18 or less carbons. Examples of the heteroatom include, for example, a nitrogen atom, an oxygen atom, a sulfur atom and a phosphorus atom. When $R^1$ is an alkyl group containing a heteroatom, the number of carbons in $R^1$ may be a total number of carbons in the carbon chain excluding the heteroatom. $R^1$ may be an alkyl group with 1 or more and 24 or less carbons not containing a heteroatom.

When $R^1$ is an alkyl group containing a heteroatom, examples of $-R^1$ include, for example, $-CH_2-(CH_2)_{n11}-Y^1-(CH_2)_{n12}-CH_3$. Here, $Y^1$ is a heteroatom, n11 and n12 each represent an integer of 0 or more and

22 or less, and a total of n11 and n12 is 0 or more and 22 or less.

In the general formula (A2), $R^{12}$ is a group with 1 or more and 24 or less carbons in total which contains a carbon atom and a heteroatom of an ion paired with $N^+$ of the pyridine ring and which may contain a heteroatom different from the heteroatom. In the compound of the general formula (A2), the ion contained in $R^{12}$ and $N^+$ of the pyridine ring form a betaine structure.

Examples of $-R^{12}$ include, for example, $-CH_2-R^{121}-Y^2-R^{122}$. Here, $Y^2$ is an anion of a heteroatom, $R^{121}$ and $R^{122}$ each represent a single bond or an alkyl group which may contain a heteroatom different from $Y^2$, and a total number of carbons in $R^{121}$ and $R^{122}$ is 0 or more and 22 or less.

Further, examples of $-R^{12}$ include, for example, $-CH_2-(CH_2)_{n21}-(Y^3)_{n22}-(CH_2)_{n23}-SO_3^-$. Here, n21 and n23 each represent an integer of 0 or more and 23 or less, a total of n21 and n23 is 0 or more and 23 or less, $Y^3$ is a heteroatom, and n22 is 0 or 1.

In the general formulas (A1) and (A2), L is n halogen atoms, n being 1, 2 or 3, and L is bonded to any n different carbon atoms at positions 2, 4 and 6 of the pyridine ring. Examples of the halogen atoms include a fluorine atom, a chlorine atom, a bromine atom and an iodine atom, and a chlorine atom is preferable. Note that the position numbers in the pyridine ring are as follows:

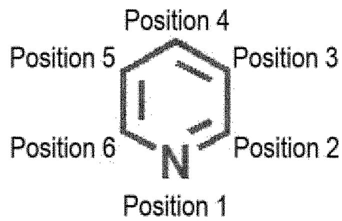


In the general formula (A1), $A^-$ is an anion. The anion of $A^-$ may be either an organic anion or an inorganic anion. Examples of the anion of $A^-$ include, for example, an anion which is a conjugate base of an acid with a pKa of 5 or less. Examples of the anion of $A^-$ include, for example, sulfonic acid ions such as a trifluoromethanesulfonate ion ($^-OTf$), a para toluenesulfonate ion ($^-OTs$), a methanesulfonate ion ($^-OMs$) and others, alkyl sulfate ions such as a methyl sulfate ion ($^-OSO_3Me$) and others, halide ions such as a chloride ion ($Cl^-$), a bromide ion ($Br^-$) and others, fluoroborate ions such as a tetrafluoroborate ion ($BF_4^-$) and others, fluorophosphate ions such as a hexafluorophosphate ion ($PF_6^-$) and others, imide ions such as a bis(trifluoromethanesulfonyl)imide ion ($(CF_3SO_2)_2N^-$) and others, fluoroacetate ions such as a trifluoroacetate ion ($CF_3COO^-$) and others, or the like. $A^-$ is preferably an anion selected from $^-OTf$, $^-OSO_3Me$, $-OTs$, $^-OMs$, $Cl^-$ and $Br^-$.

In the compound of the general formula (A1) or (A2), a carbon atom other than the carbon atom bonded to L of the carbon atoms of the pyridine ring may be bonded to a group other than L, for example, a group such as $-COO-R^2$, $-CONH-R^3$, $-CON(R^4)(R^5)$ or the like (in which $R^2$, $R^3$, $R^4$ and $R^5$ each represent an alkyl group with 3 or more carbons which may contain a heteroatom substituent).

Examples of component (A) include one or more compounds selected from a compound represented by the following general formula (A1-1) and a compound represented by the following general formula (A2-1),

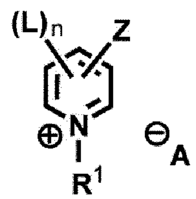

(A1-1)

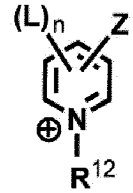

(A2-1)

wherein $R^1$ is an alkyl group with 1 or more and 24 or less carbons which may contain a heteroatom, $R^{12}$ is a group with 1 or more and 24 or less carbons in total which contains a carbon atom and a heteroatom of an ion paired with $N^+$ of the pyridine ring and which may contain a heteroatom different from the heteroatom, L is n halogen atoms, n being 1 or 2, and L is

bonded to any n different carbons at positions 2, 4 and 6 of the pyridine ring, Z is a group selected from -COO-$R^2$, -CONH-$R^3$ and-CON($R^4$)($R^5$), $R^2$, $R^3$, $R^4$ and $R^5$ each representing an alkyl group with 3 or more carbons which may contain a heteroatom, Z is bonded to a carbon atom different from the carbon atom bonded to L of the carbon atoms of the pyridine ring, $A^-$ is an anion, and a group other than L and Z may be bonded to a carbon atom other than the carbon atoms bonded to L and Z of the carbon atoms of the pyridine ring.

Specific examples or preferable examples of $R^1$, L, $A^-$ and $R^{12}$ in the general formulas (A1-1) and (A2-1) are the same as those stated in the general formulas (A1) and (A2).

In the general formulas (A1-1) and (A2-1), n is 1 or 2.

In the general formulas (A1-1) and (A2-1), Z is a group selected from -COO-$R^2$, -CONH-$R^3$ and -CON($R^4$)($R^5$), and Z is bonded to a carbon atom different from the carbon atom bonded to L of the carbon atoms of the pyridine ring. $R^2$, $R^3$, $R^4$ and $R^5$ each represent an alkyl group with 3 or more, further 8 or more and further 12 or more, and 24 or less and further 18 or less carbons which may contain a heteroatom from the viewpoint of improving oxidative decomposition activity when the composition of the present invention is used by being incorporated into oxidizing agents, bleaching agents or the like (hereinafter also simply referred to as oxidative decomposition activity). Examples of the heteroatom include, for example, a nitrogen atom, an oxygen atom, a sulfur atom and a phosphorus atom. When $R^2$, $R^3$, $R^4$ and $R^5$ each represent an alkyl group containing a heteroatom, the numbers of carbons in $R^2$, $R^3$, $R^4$ and $R^5$ may each be a total number of carbons in the carbon chain excluding the heteroatom.

In the compounds of the general formulas (A1-1) and (A2-1), a carbon atom other than the carbon atoms bonded to L and Z of the carbon atoms of the pyridine ring may be bonded to a group other than L and Z, for example, a nitro group, a trifluoromethyl group, a sulfonyl group, a cyano group, a carboxyl group, a methoxy group, a dimethylamino group or the like.

Further, examples of component (A) include a compound represented by (A1-2),

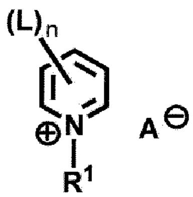

( A1-2 )

wherein $R^1$ is an alkyl group with 1 or more and 24 or less carbons which may contain a heteroatom, L is n halogen atoms, n being 1, 2 or 3, and L is bonded to any n different carbon atoms at positions 2, 4 and 6 of the pyridine ring, and $A^-$ is an anion.

Specific examples or preferable examples of $R^1$, L and $A^-$ in the general formula (A1-2) are the same as those stated in the general formula (A1).

Further, examples of component (A) include a compound represented by (A2-2),

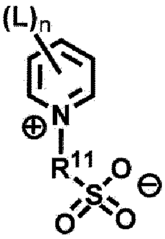

( A2-2 )

wherein L is n halogen atoms, n being 1, 2 or 3, and L is bonded to any n different carbon atoms at positions 2, 4 and 6 of the pyridine ring, and $R^{11}$ is an alkylene group with 1 or more and 24 or less carbons which may contain a heteroatom.

Specific examples or preferable examples of L in the general formula (A2-2) are the same as those stated in the general formulas (A1) and (A2).

In the general formula (A2-2), $R^{11}$ is an alkylene group with 1 or more and 24 or less carbons which may contain a heteroatom. $R^{11}$ has, for example, 2 or more and further 3 or more, and 20 or less, further 18 or less, further 16 or less, further 14 or less, further 12 or less, further 10 or less, further 8 or less, further 6 or less and further 4 or less carbons. Examples of the heteroatom include, for example, a nitrogen atom, an oxygen atom, a sulfur atom and a phosphorus atom. When $R^{11}$ is an alkylene group containing a heteroatom, the number of carbons in $R^{11}$ may be a total number of carbons in the carbon chain excluding the heteroatom. $R^{11}$ may be an alkylene group with 1 or more and 24 or less carbons not containing a heteroatom substituent.

Component (A) may be one or more compounds selected from the compound represented by the above general formula (A1-1), the compound represented by the above general formula (A1-2), the compound represented by the above

general formula (A2-1) and the compound represented by the above general formula (A2-2), and may further be one or more compounds selected from the compound represented by the above general formula (A1-1) and the compound represented by the above general formula (A2-2).

The composition of the present invention preferably contains component (A) in an amount of, for example, 0.01 mass% or more, further 0.1 mass% or more and further 1 mass% or more, and 20 mass% or less, further 10 mass% or less and further 5 mass% or less from the viewpoint of improving oxidative decomposition activity.

The composition of the present invention contains water. Examples of the water include ion-exchanged water, tap water and others. Water can be used in an amount for making a total of the makeup of the composition of the present invention 100 mass%. The composition of the present invention can contain water in an amount of, for example, 50 mass% or more, further 80 mass% or more and further 90 mass% or more, and 99 mass% or less, further 95 mass% or less and further 90 mass% or less.

The composition of the present invention can contain (B) hydrogen peroxide [hereinafter referred to as component (B)]. When the composition of the present invention contains component (B), the composition preferably contains component (B) in an amount of, for example, 0.01 mass% or more, further 1 mass% or more and further 3 mass% or more, and 30 mass% or less, further 20 mass% or less and further 10 mass% or less from the viewpoint of being excellent in safety.

When the composition of the present invention contains component (B), a content of component (B) relative to a content of 1 part by mole of component (A) is preferably, for example, 1 part by mole or more, further 22 parts by mole or more and further 33 parts by mole or more, and 100 parts by mole or less, further 55 parts by mole or less and further 44 parts by mole or less from the viewpoint of improving oxidative decomposition activity.

The composition of the present invention preferably contains a bleach activation aid as component (C).

An improved oxidative decomposition effect or bleaching effect is attained when component (A) of the present invention is combined with components (B) and (C). The reason for that is not wholly certain, but is inferred to be as follows.

Component (A) reacts with hydrogen peroxide under neutral conditions to form peroxy acid species, and these react with pigments, thereby leading to bleaching. Here, it is considered that there is no difference between solution systems and cloth systems in the first stage where component (A) reacts with hydrogen peroxide. On the other hand, there is a significant difference between solution systems and cloth systems in the next stage, i.e., the stage where peroxy acid species react with contaminant pigment molecules. To be more specific, it is considered that, in solution systems, contaminant pigments are uniformly dissolved in solutions, but in cloth systems, they are all present on cloth, and thus, frequency of collision of peroxy acid species with contaminant pigments is lower compared to solution systems. Therefore, stability of peroxy acid species in aqueous solutions has a significant effect on bleaching performance. On the other hand, it is known that component (C), for example, iminium salts generally used as catalysts for the Davis oxidation, receives reactive oxygen from peroxy acid species to produce stable peroxy acid species. Likewise, if component (C), for example, an iminium salt is used in the present invention, reactive oxygen of unstable peroxy acid species derived from component (A) is rearranged to component (C) to produce stable activated oxidation species, thereby enabling oxidative decomposition or bleaching of pigments present on cloth.

Examples of component (C) include a compound represented by the following general formula (C-1) [hereinafter referred to as component (C-1)],

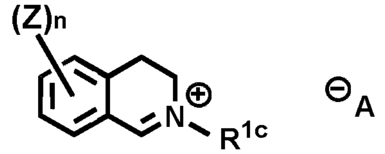

( C-1 )

wherein Z is a group selected from an alkyl group with 1 or more and 24 or less carbons which may contain a heteroatom, a nitro group, a halo group, a cyano group, an amino group, an aminoalkyl group, a thioalkyl group, a sulfoalkyl group, a carboxy ester group, a hydroxy group and a carboxyamide group, n is a number of 0 or more and 9 or less, $R^{1c}$ is an alkyl group with 1 or more and 24 or less carbons which may contain a heteroatom, and $A^-$ is an anion.

In the general formula (C-1), $A^-$ is an anion. The anion of $A^-$ may be either an organic anion or an inorganic anion. Examples of the anion of $A^-$ include, for example, an anion which is a conjugate base of an acid with a pKa of 5 or less. Examples of the anion of $A^-$ include, for example, sulfonic acid ions such as a trifluoromethanesulfonate ion ($^-$OTf), a para toluenesulfonate ion ($^-$OTs), a methanesulfonate ion ($^-$OMs) and others, alkyl sulfate ions such as a methyl sulfate ion ($^-OSO_3Me$) and others, halide ions such as a chloride ion ($Cl^-$), a bromide ion ($Br^-$) and others, fluoroborate ions such as a tetrafluoroborate ion ($BF_4^-$) and others, fluorophosphate ions such as a hexafluorophosphate ion ($PF_6^-$) and others, imide ions such as a bis(trifluoromethanesulfonyl)imide ion ($(CF_3SO_2)_2N^-$) and others, fluoroacetate ions such as a trifluoroacetate ion ($CF_3COO^-$) and others, or the like. $A^-$ is preferably an anion selected from $^-$OTf, $^-OSO_3Me$, $^-$OTs, $^-$OMs, $BF_4^-$, $Cl^-$ and $Br^-$.

From the viewpoint of improving bleaching activity, component (C-1) is preferably a quaternary imine salt, more specifically a substituted or unsubstituted isoquinolinium salt, more preferably a 3,4-dihydroisoquinolinium salt, further preferably an N-methyl-3,4-dihydroisoquinolinium salt, furthermore preferably N-methyl-3,4-dihydroisoquinolinium p-toluenesulfonate and N-methyl-3,4-dihydroisoquinolinium tetrafluoroborate, and furthermore preferably N-methyl-3,4-dihydroisoquinolinium tetrafluoroborate.

When the composition of the present invention contains component (C) and further component (C-1), the composition can contain component (C) and further component (C-1) in an amount of, for example, 0.01 mass% or more, further 0.1 mass% or more and further 0.5 mass% or more, and 20 mass% or less, further 10 mass% or less and further 5 mass% or less.

When the composition of the present invention contains component (C) and further component (C-1), a content of component (C) and further component (C-1) relative to a content of 1 part by mole of component (A) is preferably, for example, 0.01 parts by mole or more, further 0.1 parts by mole or more and further 1 part by mole or more, and 100 parts by mole or less, further 10 parts by mole or less and further 2 parts by mole or less from the viewpoint of improving bleaching activity.

The composition of the present invention preferably contains components (B) and (C). When the composition of the present invention contains component (B) and component (C) and further component (C-1), a content of component (B) relative to a content of 1 part by mole of component (C) and further component (C-1) may be, for example, 1 part by mole or more, further 22 parts by mole or more and further 33 parts by mole or more, and 100 parts by mole or less, further 55 parts by mole or less and further 44 parts by mole or less from the viewpoint of improving bleaching activity.

The composition of the present invention can contain optional components other than components (B) and (C). Examples of such optional components include, for example, chelating agents, thickeners, surfactants, polymers and others. Note that component (B) may be combined as a hydrogen peroxide solution. When a predetermined component is combined as an aspect containing water like a hydrogen peroxide solution, water contained in the aspect forms part or all of a water component in the liquid composition of the present invention.

The composition of the present invention is suitable for use in oxidizing agents. In other words, for example, the composition of the present invention can be used as an oxidizing agent together with hydrogen peroxide, or used for the production of oxidizing agents.

Further, the composition of the present invention is suitable for use in bleaching agents. In other words, for example, the composition of the present invention can be used as a bleaching agent together with hydrogen peroxide, or used for the production of bleaching agents.

A concentration of component (A) when the composition of the present invention is used, for example, when the composition is used as an oxidizing agent or a bleaching agent may be, for example, 0.0001 mass% or more, further 0.001 mass% or more and further 0.01 mass% or more, and 10 mass% or less, further 1 mass% or less and further 0.1 mass% or less.

Further, when the composition of the present invention contains component (B), a concentration of component (B) when the composition is used, for example, when the composition is used as an oxidizing agent or a bleaching agent may be, for example, 0.001 mass% or more, further 0.01 mass% or more and further 0.1 mass% or more, and 30 mass% or less, further 3 mass% or less and further 1 mass% or less.

Further, when the composition of the present invention contains component (C), a concentration of component (C) when the composition is used, for example, when the composition is used as an oxidizing agent or a bleaching agent may be, for example, 0.0001 mass% or more, further 0.001 mass% or more and further 0.01 mass% or more, and 10 mass% or less, further 1 mass% or less and further 0.1 mass% or less.

The composition of the present invention may be in any form such as a liquid composition, a gel composition or the like. The composition of the present invention is preferably in liquid form. Note that the composition of the present invention can also be obtained, for example, by mixing a solid agent containing component (A) with water. In other words, the present invention provides a kit for an acidic composition containing component (A) and water, the kit containing a solid agent containing component (A) .

The composition of the present invention may have a pH of, for example, 2 or more and further 3 or more, and less than 7, further 6 or less and further 5 or less at 25°C. The composition can contain a pH adjuster to have such a pH. Examples of the pH adjuster include hydrogen phosphates such as disodium hydrogen phosphate, potassium dihydrogen phosphate and others, organic acids such as lactic acid, succinic acid, gluconic acid, citric acid, acetic acid, tartaric acid and others, inorganic acids such as phosphoric acid, boric acid, hydrochloric acid, sulfuric acid and others, sulfonic acids such as para toluenesulfonic acid, camphorsulfonic acid, methanesulfonic acid and others, or the like. pH adjusters may be selected in combination to have a buffer capacity.

The composition of the present invention can be used for various applications such as bleaching of hard articles in bathrooms, toilets, kitchens and others, mold removal, bleaching of clothing, decomposition of substances causative of stains or odor, disinfection, virus killing, bleaching of pulp and others where oxidizing action produces useful effects on the treated objects. The composition of the present invention can be used as, for example, a bleaching agent composition, a

bleaching cleaning agent composition, a mold removing agent composition, a decolorizing agent composition, a deodorizing agent composition, a sterilizing agent composition, a virus killing agent composition or the like in combination with hydrogen peroxide or the like as necessary. Especially, the composition of the present invention is preferably used for bleaching or bleaching cleaning. Further, the composition is preferably used for bleaching or bleaching cleaning of hard articles.

The present invention provides use of the composition of the present invention for the production of oxidizing agents.

Further, the present invention provides use of the composition of the present invention for the production of bleaching agents.

If the composition of the present invention is of composition suitable for oxidation or bleaching, it is also possible to use the composition as-is to produce an oxidizing agent or a bleaching agent.

In the above use, the oxidizing agents or bleaching agents preferably contain component (A) in an amount of, for example, 0.0001 mass% or more, further 0.001 mass% or more and further 0.01 mass% or more, and 10 mass% or less, further 1 mass% or less and further 0.1 mass% or less from the viewpoint of improving oxidative decomposition activity. The composition can contain component (A) in an amount falling within this range.

In the above use, the oxidizing agents or bleaching agents preferably contain component (B). In that case, the oxidizing agents or bleaching agents preferably contain component (B) in an amount of, for example, 0.001 mass% or more, further 0.01 mass% or more and further 0.1 mass% or more, and 30 mass% or less, further 3 mass% or less and further 1 mass% or less. The composition can contain component (B) in an amount falling within this range.

In the above use, the oxidizing agents or bleaching agents can contain component (C). In that case, the oxidizing agents or bleaching agents preferably contain component (C) in an amount of, for example, 0.0001 mass% or more, further 0.001 mass% or more and further 0.01 mass% or more, and 10 mass% or less, further 1 mass% or less and further 0.1 mass% or less. Further, the oxidizing agents or bleaching agents preferably contain component (C) such that a part by mole of component (C) relative to 1 part by mole of component (A) and a part by mole of component (A) relative to 1 part by mole of component (C) fall within their respective ranges described earlier. The composition can contain component (C) in an amount falling within this range.

[Oxidizing method and bleaching method]

The present invention provides an oxidizing method including, bringing a treatment liquid prepared from the composition of the present invention into contact with an object in the presence of hydrogen peroxide.

Further, the present invention provides a bleaching method including, bringing the treatment liquid prepared from the composition of the present invention (hereinafter also referred to as the treatment liquid of the present invention) into contact with an object in the presence of hydrogen peroxide.

The treatment liquid of the present invention is preferably a treatment liquid containing components (A) and (B) and water. A treatment liquid containing components (A), (B) and (C) and water is also preferable as the treatment liquid of the present invention. If the composition of the present invention is of composition suitable for oxidation or bleaching, it is also possible to use the composition as-is as the treatment liquid of the present invention. Further, the treatment liquid of the present invention may be prepared by mixing a composition containing component (A) and not containing component (B) with a composition containing a compound which is a supply source for component (B) or component (B). When component (C) is used, it may be mixed anyhow, and component (C) may be contained in one of or both the above two compositions.

The treatment liquid of the present invention can be obtained by mixing the composition of the present invention containing component (A) and water with component (B), and/or a supply source for component (B), for example, a peroxide which produces hydrogen peroxide in water, and as necessary, water, component (C) and others. The peroxide which produces hydrogen peroxide in water is exemplified by an inorganic peroxide or a hydrogen peroxide adduct, and is preferably a percarbonate, a tripolyphosphate/hydrogen peroxide adduct, a pyrophosphate/hydrogen peroxide adduct, urea/hydrogen peroxide adduct, a sulfate/hydrogen peroxide adduct, a perborate, a persilicate or a peroxide salt and more preferably sodium percarbonate, sodium tripolyphosphate/hydrogen peroxide adduct, sodium pyrophosphate/hydrogen peroxide adduct, urea/hydrogen peroxide adduct, or $4Na_2SO_4/2H_2O_2$, sodium perborate monohydrate, sodium perborate tetrahydrate, sodium persilicate, sodium peroxide, calcium peroxide or the like. Further, the treatment liquid can also be obtained by preparing the composition of the present invention using a kit as described above, and thereafter mixing the composition with component (B) and/or a supply source for component (B), and as necessary, water, component (C) and others. Further, as described later, depending on the use purpose, a pH adjuster, for example, one also functioning as a buffering agent like a phosphate buffer solution may be added to adjust a pH of the treatment liquid to, for example, 3 or more, further 5 or more and further 6 or more, and 12 or less, further 10 or less and further 8 or less.

The treatment liquid of the present invention preferably contains component (A) in an amount of, for example, 0.0001 mass% or more, further 0.001 mass% or more and further 0.01 mass% or more, and 10 mass% or less, further 1

mass% or less and further 0.1 mass% or less from the viewpoint of improving oxidative decomposition activity.

The treatment liquid of the present invention can contain component (B) in an amount of, for example, 0.001 mass% or more, further 0.01 mass% or more and further 0.1 mass% or more, and 30 mass% or less, further 3 mass% or less and further 1 mass% or less.

The treatment liquid of the present invention can contain component (C) in an amount of, for example, 0.0001 mass% or more, further 0.001 mass% or more and further 0.01 mass% or more, and 10 mass% or less, further 1 mass% or less and further 0.1 mass% or less from the viewpoint of improving oxidative decomposition activity.

Examples of the object in the oxidizing method and the bleaching method include, for example, hard articles in bathrooms, toilets, kitchens and others, textile products such as clothing and others, or the like. In the oxidizing method and the bleaching method, the composition of the present invention can be brought into contact with the object in the presence of hydrogen peroxide by a method such as immersing, applying, spraying or the like. A contact time or a contact temperature when the composition of the present invention is brought into contact with the object is not limited.

The treatment liquid of the present invention can contain the optional components stated in the composition of the present invention.

The treatment liquid of the present invention may have a pH of, for example, 3 or more, further 5 or more and further 6 or more, and 12 or less, further 10 or less and further 8 or less. This pH is a pH at a temperature at which the oxidizing method or the bleaching method of the present invention is carried out, and may be, for example, a pH at 25°C. In the oxidizing method or the bleaching method of the present invention, excellent oxidizing power or bleaching power can be obtained even at a pH near neutral in the presence of hydrogen peroxide.

[Storing method]

The present invention provides a method for storing a compound including, storing component (A) in an acidic solution (hereinafter also referred to as the storing method of the present invention).

The matters stated in the composition of the present invention can be appropriately applied to the storing method of the present invention. Specific examples or preferable examples of component (A) and others in the storing method of the present invention are also the same as in the composition of the present invention. The storing method of the present invention may be a method for storing component (A) or the composition of the present invention.

In the storing method of the present invention, a content of component (A) in the acidic solution may be, for example, 0.01 mass% or more, further 0.1 mass% or more and further 1 mass% or more, and 20 mass% or less, further 10 mass% or less and further 5 mass% or less.

In the storing method of the present invention, component (A) is stored in the acidic solution. The acidic solution used for storage may have a pH of, for example, 2 or more and further 3 or more, and less than 7, further 6 or less and further 5 or less. The acidic solution can contain a pH adjuster to have such a pH. Examples of the pH adjuster include hydrogen phosphates such as disodium hydrogen phosphate, potassium dihydrogen phosphate and others, organic acids such as lactic acid, succinic acid, gluconic acid, citric acid, acetic acid, tartaric acid and others, inorganic acids such as phosphoric acid, boric acid, hydrochloric acid, sulfuric acid and others, sulfonic acids such as para toluenesulfonic acid, camphorsulfonic acid, methanesulfonic acid and others, or the like. pH adjusters may be selected in combination to have a buffer capacity. Note that this pH is a pH in a state where component (A) and optional components such as component (B) and others are contained.

The storing method of the present invention can be carried out under the coexistence of components other than component (A). For example, storing can be carried out in the acidic solution with the optional components stated in the composition of the present invention added thereto.

In the present invention, component (A) is preferably stored in the acidic solution under the coexistence of hydrogen peroxide of component (B). When component (B) is made to coexist in the storing method of the present invention, a content of component (B) in the acidic solution may be, for example, 0.01 mass% or more, further 1 mass% or more and further 3 mass% or more, and 30 mass% or less, further 20 mass% or less and further 10 mass% or less.

Further, in the present invention, component (A) is preferably stored in the acidic solution under the coexistence of the bleach activation aid of component (C). When component (C) is made to coexist in the storing method of the present invention, a content of component (C) in the acidic solution may be, for example, 0.01 mass% or more, further 0.1 mass% or more and further 1 mass% or more, and 20 mass% or less, further 10 mass% or less and further 5 mass% or less.

The storing method of the present invention can be used as a method for storing, for example, the composition of the present invention, and further, the composition of the present invention used for oxidizing agents or the composition of the present invention used for bleaching agents.

In the storing method of the present invention, a temperature of the acidic solution is not particularly limited, but may be, for example, 0°C or more and further 25°C or more, and 60°C or less and further 50°C or less.

In the storing method of the present invention, a storage period is not particularly limited, but may be, for example, 1 day or more and further 14 days or more, and 730 days or less and further 365 days or less.

In the storing method of the present invention, component (A) can be stored in such a manner that, for example, water and component (A), and as necessary, component (B), component (C) and other optional components are put into a predetermined container, and the solution is adjusted to be acidic in nature and preferably adjusted to have a pH falling within the above range. A shape, capacity or the like of the container is not limited. The container preferably has a sealing member for an opening, such as a lid, a stopper or the like.

In addition to the aforementioned embodiments, the present invention discloses the aspects below.

<1> A composition containing, (A) one or more compounds selected from a compound represented by the following general formula (A1) and a compound represented by the following general formula (A2) [hereinafter referred to as component (A)] and water, the composition being acidic,

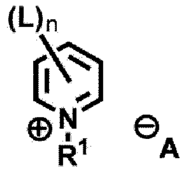

(A1)

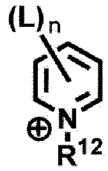

(A2)

wherein $R^1$ is an alkyl group with 1 or more and 24 or less carbons which may contain a heteroatom, $R^{12}$ is a group with 1 or more and 24 or less carbons in total which contains a carbon atom and a heteroatom of an ion paired with $N^+$ of the pyridine ring and which may contain a heteroatom different from the heteroatom, L is n halogen atoms, n being 1, 2 or 3, and L is bonded to any n different carbon atoms at positions 2, 4 and 6 of the pyridine ring, $A^-$ is an anion, and a group other than L may be bonded to a carbon atom other than the carbon atom bonded to L of the carbon atoms of the pyridine ring.

<2> The composition according to <1>, wherein in the general formula (A1), the alkyl group of $R^1$ has 1 or more, further 8 or more and further 12 or more, and 24 or less, further 20 or less and further 18 or less carbons.

<3> The composition according to <1> or <2>, wherein in the general formula (A1), the heteroatom of $R^1$ is at least one selected from a nitrogen atom, an oxygen atom, a sulfur atom and a phosphorus atom.

<4> The composition according to any of <1> to <3>, wherein in the general formula (A1), $R^1$ is an alkyl group with 1 or more and 24 or less carbons not containing a heteroatom.

<5> The composition according to any of <1> to <3>, wherein in the general formula (A1), $R^1$ is an alkyl group containing a heteroatom, and further $-CH_2-(CH_2)_{n11}-Y^1-(CH_2)_{n12}-CH_3$, in which $Y^1$ is a heteroatom, n11 and n12 each represent an integer of 0 or more and 22 or less, and a total of n11 and n12 is 0 or more and 22 or less.

<6> The composition according to any of <1> to <5>, wherein in the general formula (A2), $-R^{12}$ is $-CH_2-R^{121}-Y^2-R^{122}$, $Y^2$ is an anion of a heteroatom, $R^{121}$ and $R^{122}$ each represent a single bond or an alkyl group which may contain a heteroatom different from $Y^2$, and a total number of carbons in $R^{121}$ and $R^{122}$ is 0 or more and 22 or less.

<7> The composition according to any of <1> to <5>, wherein in the general formula (A2), $-R^{12}$ is $-CH_2-(CH_2)_{n21}-(Y^3)_{n22}-(CH_2)_{n23}-SO_3^-$, n21 and n23 each represent an integer of 0 or more and 23 or less, a total of n21 and n23 is 0 or more and 23 or less, $Y^3$ is a heteroatom, and n22 is 0 or 1.

<8> The composition according to any of <1> to <7>, wherein in the general formula (A1) or the general formula (A2), L is a halogen atom selected from a fluorine atom, a chlorine atom, a bromine atom and an iodine atom, and further a chlorine atom.

<9> The composition according to any of <1> to <8>, wherein in the general formula (A1), $A^-$ is an anion selected from an organic anion and an inorganic anion.

<10> The composition according to any of <1> to <9>, wherein in the general formula (A1), $A^-$ is an anion which is a conjugate base of an acid with a pKa of 5 or less.

<11> The composition according to any of <1> to <10>, wherein in the general formula (A1), $A^-$ is an anion selected from a sulfonic acid ion, an alkyl sulfate ion, a halide ion, a fluoroborate ion, a fluorophosphate ion, an imide ion and a fluoroacetate ion, further an anion selected from a trifluoromethanesulfonate ion ($^-OTf$), a para toluenesulfonate ion ($^-OTs$), a methanesulfonate ion ($^-OMs$), a methyl sulfate ion ($^-OSO_3Me$), a chloride ion ($Cl^-$), a bromide ion ($Br^-$), a tetrafluoroborate ion ($BF_4^-$), a hexafluorophosphate ion ($PF_6^-$), a bis(trifluoromethanesulfonyl)imide ion ($(CF_3SO_2)_2N^-$) and a trifluoroacetate ion ($CF_3COO^-$), and further an anion selected from $^-OTf$, $^-OSO_3Me$, $^-OTs$,

$^-OMs$, $Cl^-$ and $Br^-$.
<12> The composition according to any of <1> to <11>, wherein in the general formula (A1) or the general formula (A2), a carbon atom other than the carbon atom bonded to L of the carbon atoms of the pyridine ring is bonded to a group other than L, and further, a carbon atom other than the carbon atom bonded to L of the carbon atoms of the pyridine ring is bonded to a group selected from -COO-$R^2$, -CONH-$R^3$ and -CON($R^4$)($R^5$) (in which $R^2$, $R^3$, $R^4$ and $R^5$ each represent an alkyl group with 3 or more carbons which may contain a heteroatom substituent).
<13> The composition according to any of <1> to <12>, wherein component (A) is one or more compounds selected from a compound represented by the following general formula (A1-1), a compound represented by the following general formula (A1-2), a compound represented by the following general formula (A2-1) and a compound represented by the following general formula (A2-2), further one or more compounds selected from the compound represented by the following general formula (A1-1), the compound represented by the following general formula (A1-2) and the compound represented by the following general formula (A2-2), and further one or more compounds selected from the compound represented by the following general formula (A1-1) and the compound represented by the following general formula (A2-2),

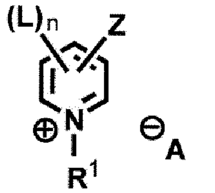

(A1-1)

wherein in the formula (A1-1), $R^1$ is an alkyl group with 1 or more and 24 or less carbons which may contain a heteroatom, L is n halogen atoms, n being 1 or 2, and L is bonded to any n different carbons at positions 2, 4 and 6 of the pyridine ring, Z is a group selected from -COO-$R^2$, -CONH-$R^3$ and -CON($R^4$)($R^5$), $R^2$, $R^3$, $R^4$ and $R^5$ each representing an alkyl group with 3 or more carbons which may contain a heteroatom, Z is bonded to a carbon atom different from the carbon atom bonded to L of the carbon atoms of the pyridine ring, A- is an anion, and a group other than L and Z may be bonded to a carbon atom other than the carbon atoms bonded to L and Z of the carbon atoms of the pyridine ring,

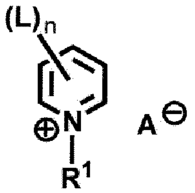

(A1-2)

wherein in the formula (A1-2), $R^1$ is an alkyl group with 1 or more and 24 or less carbons which may contain a heteroatom, L is n halogen atoms, n being 1, 2 or 3, and L is bonded to any n different carbon atoms at positions 2, 4 and 6 of the pyridine ring, and $A^-$ is an anion,

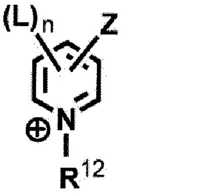

(A2-1)

wherein in the formula (A2-1), $R^{12}$ is a group with 1 or more and 24 or less carbons in total which contains a carbon atom and a heteroatom of an ion paired with $N^+$ of the pyridine ring and which may contain a heteroatom different from the heteroatom, L is n halogen atoms, n being 1 or 2, and L is bonded to any n different carbons at positions 2, 4 and 6 of the pyridine ring, Z is a group selected from -COO-$R^2$, -CONH-$R^3$ and -CON($R^4$)($R^5$), $R^2$, $R^3$, $R^4$ and $R^5$ each representing an alkyl group with 3 or more carbons which may contain a heteroatom, Z is bonded to a carbon atom different from the carbon atom bonded to L of the carbon atoms of the pyridine ring, $A^-$ is an anion, and a group other than L and Z may be bonded to a carbon atom other than the carbon atoms bonded to L and Z of the carbon atoms of the pyridine ring, and

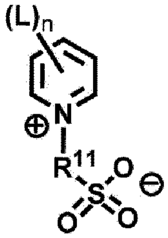

( A2-2 )

wherein in the formula (A2-2), $R^{11}$ is an alkylene group with 1 or more and 24 or less carbons which may contain a heteroatom, L is n halogen atoms, n being 1 or 2, and L is bonded to any n different carbons at positions 2, 4 and 6 of the pyridine ring, and a group other than L may be bonded to a carbon atom other than the carbon atom bonded to L of the carbon atoms of the pyridine ring.

<14> The composition according to <13>, wherein in the general formula (A1-1) or the general formula (A1-2), the alkyl group of $R^1$ has 1 or more, further 8 or more and further 12 or more, and 24 or less, further 20 or less and further 18 or less carbons.

<15> The composition according to <13> or <14>, wherein in the general formula (A1-1) or the general formula (A1-2), the heteroatom of $R^1$ is at least one selected from a nitrogen atom, an oxygen atom, a sulfur atom and a phosphorus atom.

<16> The composition according to any of <13> to <15>, wherein in the general formula (A1-1) or the general formula (A1-2), $R^1$ is an alkyl group with 1 or more and 24 or less carbons not containing a heteroatom.

<17> The composition according to any of <13> to <15>, wherein in the general formula (A1-1) or the general formula (A1-2), $R^1$ is an alkyl group containing a heteroatom, and further $-CH_2-(CH_2)_{n11}-Y^1-(CH_2)_{n12}-CH_3$, in which $Y^1$ is a heteroatom, n11 and n12 each represent an integer of 0 or more and 22 or less, and a total of n11 and n12 is 0 or more and 22 or less.

<18> The composition according to any of <13> to <17>, wherein in the general formula (A2-1), $-R^{12}$ is $-CH_2-R^{121}-Y^2-R^{122}$, $Y^2$ is an anion of a heteroatom, $R^{121}$ and $R^{122}$ each represent a single bond or an alkyl group which may contain a heteroatom different from $Y^2$, and a total number of carbons in $R^{121}$ and $R^{122}$ is 0 or more and 22 or less.

<19> The composition according to any of <13> to <17>, wherein in the general formula (A2-1), $-R^{12}$ is $-CH_2-(CH_2)_{n21}-(Y^3)_{n22}-(CH_2)_{n23}-SO_3^-$, n21 and n23 each represent an integer of 0 or more and 23 or less, a total of n21 and n23 is 0 or more and 23 or less, $Y^3$ is a heteroatom, and n22 is 0 or 1.

<20> The composition according to any of <13> to <19>, wherein in the general formula (A1-1), the general formula (A1-2), the general formula (A2-1) or the general formula (A2-2), L is a halogen atom selected from a fluorine atom, a chlorine atom, a bromine atom and an iodine atom, and further a chlorine atom.

<21> The composition according to any of <13> to <20>, wherein in the general formula (A1-1) or the general formula (A1-2), $A^-$ is an anion selected from an organic anion and an inorganic anion.

<22> The composition according to any of <13> to <21>, wherein in the general formula (A1-1) or the general formula (A1-2), $A^-$ is an anion which is a conjugate base of an acid with a pKa of 5 or less.

<23> The composition according to any of <13> to <22>, wherein in the general formula (A1-1) or the general formula (A1-2), $A^-$ is an anion selected from a sulfonic acid ion, an alkyl sulfate ion, a halide ion, a fluoroborate ion, a fluorophosphate ion, an imide ion and a fluoroacetate ion, further an anion selected from a trifluoromethanesulfonate ion ($^-OTf$), a para toluenesulfonate ion ($^-OTs$), a methanesulfonate ion ($^-OMs$), a methyl sulfate ion ($^-OSO_3Me$), a chloride ion ($Cl^-$), a bromide ion ($Br^-$), a tetrafluoroborate ion ($BF_4^-$), a hexafluorophosphate ion ($PF_6^-$), a bis(trifluoromethanesulfonyl)imide ion ($(CF_3SO_2)_2N^-$) and a trifluoroacetate ion ($CF_3COO^-$), and further an anion selected from $^-OTf$, $^-OSO_3Me$, $^-OTs$, $^-OMs$, $Cl^-$ and $Br^-$.

<24> The composition according to any of <13> to <23>, wherein in the general formula (A1-1) or the general formula (A2-1), a carbon atom other than the carbon atom bonded to L of the carbon atoms of the pyridine ring is bonded to a group other than L, and further, a carbon atom other than the carbon atom bonded to L of the carbon atoms of the pyridine ring is bonded to a group selected from $-COO-R^2$, $-CONH-R^3$ and $-CON(R^4)(R^5)$ (in which $R^2$, $R^3$, $R^4$ and $R^5$ each represent an alkyl group with 3 or more carbons which may contain a heteroatom substituent).

<25> The composition according to any of <13> to <24>, wherein in the general formula (A2-2), $R^{11}$ has 2 or more and further 3 or more, and 20 or less, further 18 or less, further 16 or less, further 14 or less, further 12 or less, further 10 or less, further 8 or less, further 6 or less and further 4 or less carbons.

<26> The composition according to any of <13> to <25>, wherein in the general formula (A2-2), $R^{11}$ contains a heteroatom, and the heteroatom is at least one selected from a nitrogen atom, an oxygen atom, a sulfur atom and a phosphorus atom.

<27> The composition according to any of <13> to <25>, wherein in the general formula (A2-2), $R^{11}$ is an alkylene

group with 1 or more and 24 or less carbons not containing a heteroatom substituent.

<28> The composition according to any of <13> to <27>, wherein in the general formula (A1-1), $R^1$ is a methyl group, L is a chlorine atom, n is 1, L is bonded to a carbon atom at position 2 of the pyridine ring, Z is a group selected from -COO-$R^2$ and -CONH-$R^3$, Z being bonded at position 3 of the pyridine ring, and $A^-$ is $^-$OTf.

<29> The composition according to any of <13> to <28>, wherein in the general formula (A1-1), $R^1$ is a methyl group, L is a chlorine atom, n is 1, L is bonded to a carbon atom at position 2 of the pyridine ring, Z is-COO-$R^2$, Z being bonded at position 3 of the pyridine ring, $R^2$ is a butyl group, and $A^-$ is $^-$OTf.

<30> The composition according to any of <13> to <29>, wherein in the general formula (A1-1), $R^1$ is a methyl group, L is a chlorine atom, n is 1, L is bonded to a carbon atom at position 2 of the pyridine ring, Z is - CONH-$R^3$, Z being bonded at position 3 of the pyridine ring, $R^3$ is a butyl group, and $A^-$ is $^-$OTf.

<31> The composition according to any of <13> to <30>, wherein in the general formula (A1-2), $R^1$ is a dodecyl group, L is a chlorine atom, n is 1, L is bonded to a carbon atom at position 2 of the pyridine ring, and $A^-$ is $^-$OTf.

<32> The composition according to any of <13> to <31>, wherein in the general formula (A2-2), $R^{11}$ is an alkylene group with 3 carbons, L is a chlorine atom, n is 1, L is bonded to a carbon atom at position 2 of the pyridine ring, and $A^-$ is $^-$OTf.

<33> The composition according to any of <1> to <32>, containing component (A) in an amount of 0.01 mass% or more, further 0.1 mass% or more and further 1 mass% or more, and 20 mass% or less, further 10 mass% or less and further 5 mass% or less.

<34> The composition according to any of <1> to <33>, containing water in an amount of 50 mass% or more, further 80 mass% or more and further 90 mass% or more, and 99 mass% or less, further 95 mass% or less and further 90 mass% or less.

<35> The composition according to any of <1> to <30>, further containing (B) hydrogen peroxide [hereinafter referred to as component (B)].

<36> The composition according to <35>, containing component (B) in an amount of 0.01 mass% or more, further 1 mass% or more and further 3 mass% or more, and 30 mass% or less, further 20 mass% or less and further 10 mass% or less.

<37> The composition according to <35> or <36>, wherein a content of component (A) relative to 1 part by mole of component (B) is further 22 parts by mole or more and further 33 parts by mole or more, and 100 parts by mole or less, further 55 parts by mole or less and further 44 parts by mole or less.

<38> The composition according to any of <1> to <37>, further containing (C) a bleach activation aid [hereinafter referred to as component (C)].

<39> The composition according to <38>, wherein component (C) is a compound represented by the following general formula (C-1):

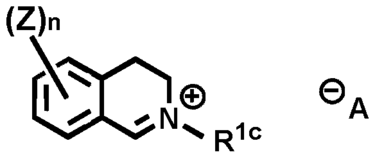

(C-1)

wherein Z is a group selected from an alkyl group with 1 or more and 24 or less carbons which may contain a heteroatom, a nitro group, a halo group, a cyano group, an amino group, an aminoalkyl group, a thioalkyl group, a sulfoalkyl group, a carboxy ester group, a hydroxy group and a carboxyamide group, n being a number of 0 or more and 9 or less, $R^{1c}$ is an alkyl group with 1 or more and 24 or less carbons which may contain a heteroatom, and $A^-$is an anion.

<40> The composition according to <39>, wherein in the general formula (C-1), n is 0, $R^{1c}$ is a methyl group, and $A^-$ is $BF_4^-$.

<41> The composition according to <39> or <40>, containing component (C) in an amount of 0.01 mass% or more, further 0.1 mass% or more and further 1 mass% or more, and 20 mass% or less, further 10 mass% or less and further 5 mass% or less.

<42> The composition according to any of <39> to <41>, wherein a content of component (C) relative to a content of 1 part by mole of component (A) is 0.01 parts by mole or more, further 0.1 parts by mole or more and further 1 part by mole or more, and 100 parts by mole or less, further 10 parts by mole or less and further 2 parts by mole or less.

<43> The composition according to any of <38> to <42>, wherein the composition contains components (B) and (C), and a content of component (B) relative to a content of 1 part by mole of component (C) is 1 part by mole or more, further 22 parts by mole or more and further 33 parts by mole or more, and 100 parts by mole or less, further 55 parts by mole or less and further 44 parts by mole or less.

<44> The composition according to any of <1> to <43>, wherein the composition has a pH of 2 or more and further 3 or

more, and less than 7, further 6 or less and further 5 or less at 25°C.

<45> The composition according to any of <1> to <44>, containing a pH adjuster.

<46> The composition according to <45>, wherein the pH adjuster is a pH adjuster selected from a hydrogen phosphate selected from disodium hydrogen phosphate and potassium dihydrogen phosphate, an organic acid selected from lactic acid, succinic acid, gluconic acid, citric acid, acetic acid and tartaric acid, an inorganic acid selected from phosphoric acid, boric acid, hydrochloric acid and sulfuric acid, and a sulfonic acid selected from para toluenesulfonic acid, camphorsulfonic acid and methanesulfonic acid.

<47> The composition according to any of <1> to <46>, wherein the composition is used for oxidizing agents.

<48> The composition according to any of <1> to <46>, wherein the composition is used for bleaching agents.

<49> An oxidizing method including, bringing a treatment liquid prepared from the composition according to any of <1> to <48> into contact with an object in the presence of hydrogen peroxide.

<50> A bleaching method including, bringing a treatment liquid prepared from the composition according to any of <1> to <48> into contact with an object in the presence of hydrogen peroxide.

<51> The method according to <49> or <50>, wherein the treatment liquid contains components (A) and (B) and water.

<52> The method according to <51>, wherein the treatment liquid contains component (B) in an amount of 0.001 mass% or more, further 0.01 mass% or more and further 0.1 mass% or more, and 30 mass% or less, further 3 mass% or less and further 1 mass% or less.

<53> The method according to any of <49> to <52>, wherein the treatment liquid contains component (A) in an amount of 0.0001 mass% or more, further 0.001 mass% or more and further 0.01 mass% or more, and 10 mass% or less, further 1 mass% or less and further 0.1 mass% or less.

<54> The method according to any of <49> to <53>, wherein the treatment liquid contains water in an amount of 80 mass% or more, further 90 mass% or more and further 95 mass% or more, and 99.99 mass% or less, further 98 mass% or less and further 95 mass% or less.

<55> The method according to any of <49> to <54>, wherein the treatment liquid has a pH of 3 or more, further 5 or more and further 6 or more, and 12 or less, further 10 or less and further 8 or less.

<56> A method for storing a compound including, storing (A) one or more compounds selected from a compound represented by the following general formula (A1) and a compound represented by the following general formula (A2) [hereinafter referred to as component (A)] in an acidic solution,

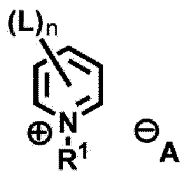

(A1)

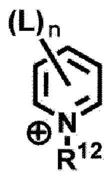

(A2)

wherein $R^1$ is an alkyl group with 1 or more and 24 or less carbons which may contain a heteroatom, $R^{12}$ is a group with 1 or more and 24 or less carbons in total which contains a carbon atom and a heteroatom of an ion paired with $N^+$ of the pyridine ring and which may contain a heteroatom different from the heteroatom, L is n halogen atoms, n being 1, 2 or 3, and L is bonded to any n different carbon atoms at positions 2, 4 and 6 of the pyridine ring, $A^-$ is an anion, and a group other than L may be bonded to a carbon atom other than the carbon atom bonded to L of the carbon atoms of the pyridine ring.

<57> The method for storing a compound according to <56>, wherein a content of component (A) in the solution is 0.01 mass% or more, further 0.1 mass% or more and further 1 mass% or more, and 20 mass% or less, further 10 mass% or less and further 5 mass% or less.

<58> The method for storing a compound according to <56> or <57>, wherein component (A) is stored in a solution having a pH of 2 or more and further 3 or more, and less than 7, further 6 or less and further 5 or less.

<59> The method for storing a compound according to any of <56> to <58>, wherein the solution in which component (A) is stored contains a pH adjuster.

<60> The method for storing a compound according to <59>, wherein the pH adjuster is a pH adjuster selected from a hydrogen phosphate selected from disodium hydrogen phosphate and potassium dihydrogen phosphate, an organic acid selected from lactic acid, succinic acid, gluconic acid, citric acid, acetic acid and tartaric acid, an inorganic acid

selected from phosphoric acid, boric acid, hydrochloric acid and sulfuric acid, and a sulfonic acid selected from para toluenesulfonic acid, camphorsulfonic acid and methanesulfonic acid.

<61> The method for storing a compound according to <56> to <60>, wherein component (A) is stored under the coexistence of (B) hydrogen peroxide [hereinafter referred to as component (B)].

<62> The method for storing a compound according to <61>, wherein a content of component (B) in the solution is 0.01 mass% or more, further 1 mass% or more and further 3 mass% or more, and 30 mass% or less, further 20 mass% or less and further 10 mass% or less.

<63> The storing method according to any of <56> to <62>, wherein the storing method is a method for storing the composition according to any of <1> to <48>, and further the composition used for oxidizing agents or the composition used for bleaching agents.

<64> The storing method according to any of <56> to <63>, wherein a temperature of the acidic solution is 0°C or more and further 25°C or more, and 60°C or less and further 50°C or less.

<65> The storing method according to any of <56> to <64>, wherein a storage period is 1 day or more and further 14 days or more, and 730 days or less and further 365 days or less.

<66> Use of the composition according to any of <1> to <48> for the production of oxidizing agents.

<67> Use of the composition according to any of <1> to <48> for the production of bleaching agents.

<68> An oxidizing method or a bleaching method including, bringing a treatment liquid into contact with an object, the treatment liquid being prepared from a liquid composition containing (A) a compound represented by the following general formula (A1-1) [hereinafter referred to as component (A)], (B) hydrogen peroxide [hereinafter referred to as component (B)] and water, the liquid composition being acidic,

wherein the treatment liquid has a pH of 5 or more and 10 or less,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,

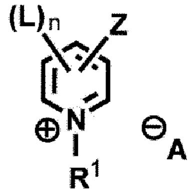

(A1-1)

wherein $R^1$ is a methyl group, L is a chlorine atom, n is 1, L is bonded to a carbon atom at position 2 of the pyridine ring, Z is $-COO-R^2$, Z being bonded to a carbon atom at position 3 of the pyridine ring, $R^2$ is a butyl group, and $A^-$ is $^-OTf$.

<69> An oxidizing method or a bleaching method including, bringing a treatment liquid into contact with an object, the treatment liquid being prepared from a liquid composition containing (A) a compound represented by the following general formula (A1-1) [hereinafter referred to as component (A)], (B) hydrogen peroxide [hereinafter referred to as component (B)] and water, the liquid composition being acidic,

wherein the treatment liquid has a pH of 5 or more and 10 or less,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,

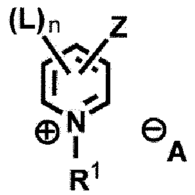

(A1-1)

wherein $R^1$ is a methyl group, L is a chlorine atom, n is 1, L is bonded to a carbon atom at position 2 of the pyridine ring, Z is $-CONH-R^3$, Z being bonded to a carbon atom at position 3 of the pyridine ring, $R^3$ is a butyl group, and $A^-$ is $^-OTf$.

<70> An oxidizing method or a bleaching method including, bringing a treatment liquid into contact with an object, the

treatment liquid being prepared from a liquid composition containing (A) a compound represented by the following general formula (A1-2) [hereinafter referred to as component (A)], (B) hydrogen peroxide [hereinafter referred to as component (B)] and water, the liquid composition being acidic,

wherein the treatment liquid has a pH of 5 or more and 10 or less,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,

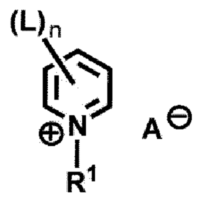

( A1-2 )

wherein $R^1$ is a dodecyl group, L is a chlorine atom, n is 1, L is bonded to a carbon atom at position 2 of the pyridine ring, and $A^-$ is $^-$OTf.

<71> An oxidizing method or a bleaching method including, bringing a treatment liquid into contact with an object, the treatment liquid being prepared from a liquid composition containing (A) a compound represented by the following general formula (A2-2) [hereinafter referred to as component (A)], (B) hydrogen peroxide [hereinafter referred to as component (B)] and water, the liquid composition being acidic,

wherein the treatment liquid has a pH of 5 or more and 10 or less,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,

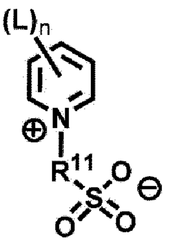

( A2-2 )

wherein $R^{11}$ is an alkylene group with 3 carbons, L is a chlorine atom, n is 1, L is bonded to a carbon atom at position 2 of the pyridine ring, and $A^-$ is $^-$OTf.

<72> A treatment liquid for oxidation or bleaching prepared from a liquid composition containing (A) a compound represented by the following general formula (A1-1) [hereinafter referred to as component (A)], (B) hydrogen peroxide [hereinafter referred to as component (B)] and water, the liquid composition being acidic,

wherein the treatment liquid has a pH of 5 or more and 10 or less at 25°C,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,

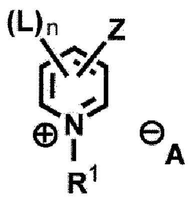

( A1-1 )

wherein $R^1$ is a methyl group, L is a chlorine atom, n is 1, L is bonded to a carbon atom at position 2 of the pyridine ring, Z

is -COO-$R^2$, Z being bonded to a carbon atom at position 3 of the pyridine ring, $R^2$ is a butyl group, and $A^-$ is $^-$OTf.

<73> A treatment liquid for oxidation or bleaching prepared from a liquid composition containing (A) a compound represented by the following general formula (A1-1) [hereinafter referred to as component (A)], (B) hydrogen peroxide [hereinafter referred to as component (B)] and water, the liquid composition being acidic,

wherein the treatment liquid has a pH of 5 or more and 10 or less at 25°C,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,

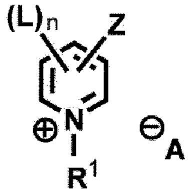

(A1-1)

wherein $R^1$ is a methyl group, L is a chlorine atom, n is 1, L is bonded to a carbon atom at position 2 of the pyridine ring, Z is -CONH-$R^3$, Z being bonded to a carbon atom at position 3 of the pyridine ring, $R^3$ is a butyl group, and $A^-$ is $^-$OTf.

<74> A treatment liquid for oxidation or bleaching prepared from a liquid composition containing (A) a compound represented by the following general formula (A1-2) [hereinafter referred to as component (A)], (B) hydrogen peroxide [hereinafter referred to as component (B)] and water, the liquid composition being acidic,

wherein the treatment liquid has a pH of 5 or more and 10 or less at 25°C,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,

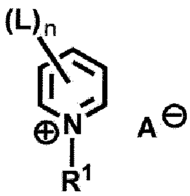

(A1-2)

wherein $R^1$ is a dodecyl group, L is a chlorine atom, n is 1, L is bonded to a carbon atom at position 2 of the pyridine ring, and $A^-$ is $^-$OTf.

<75> A treatment liquid for oxidation or bleaching prepared from a liquid composition containing (A) a compound represented by the following general formula (A2-2) [hereinafter referred to as component (A)], (B) hydrogen peroxide [hereinafter referred to as component (B)] and water, the liquid composition being acidic,

wherein the treatment liquid has a pH of 5 or more and 10 or less at 25°C,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,

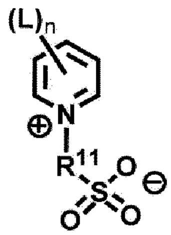

(A2-2)

wherein $R^{11}$ is an alkylene group with 3 carbons, L is a chlorine atom, n is 1, L is bonded to a carbon atom at position 2 of the pyridine ring, and $A^-$ is $^-OTf$.

<76> A treatment liquid for oxidation or bleaching containing, (A) a compound represented by the following general formula (A1-1) in an amount of 0.0001 mass% or more and 1 mass% or less, (B) hydrogen peroxide in an amount of 0.001 mass% or more and 3 mass% or less, and water in an amount of 80 mass% or more and 99.99 mass% or less, the treatment liquid having a pH of 5 or more and 10 or less at 25°C,

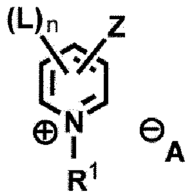

( A1-1 )

wherein $R^1$ is a methyl group, L is a chlorine atom, n is 1, L is bonded to a carbon atom at position 2 of the pyridine ring, Z is -COO-$R^2$, Z being bonded to a carbon atom at position 3 of the pyridine ring, $R^2$ is a butyl group, and $A^-$ is $^-OTf$.

<77> A treatment liquid for oxidation or bleaching containing, (A) a compound represented by the following general formula (A1-1) in an amount of 0.0001 mass% or more and 1 mass% or less, (B) hydrogen peroxide in an amount of 0.001 mass% or more and 3 mass% or less, and water in an amount of 80 mass% or more and 99.99 mass% or less, the treatment liquid having a pH of 5 or more and 10 or less at 25°C,

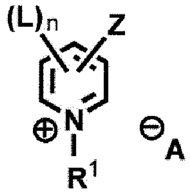

( A1-1 )

wherein $R^1$ is a methyl group, L is a chlorine atom, n is 1, L is bonded to a carbon atom at position 2 of the pyridine ring, Z is -CONH-$R^3$, Z being bonded to a carbon atom at position 3 of the pyridine ring, $R^3$ is a butyl group, and $A^-$ is $^-OTf$.

<78> A treatment liquid for oxidation or bleaching containing, (A) a compound represented by the following general formula (A1-2) in an amount of 0.0001 mass% or more and 1 mass% or less, (B) hydrogen peroxide in an amount of 0.001 mass% or more and 3 mass% or less, and water in an amount of 80 mass% or more and 99.99 mass% or less, the treatment liquid having a pH of 5 or more and 10 or less at 25°C,

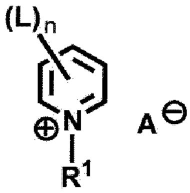

( A1-2 )

wherein $R^1$ is a dodecyl group, L is a chlorine atom, n is 1, L is bonded to a carbon atom at position 2 of the pyridine ring, and $A^-$ is $^-OTf$.

<79> A treatment liquid for oxidation or bleaching containing, (A) a compound represented by the following general formula (A2-2) in an amount of 0.0001 mass% or more and 1 mass% or less, (B) hydrogen peroxide in an amount of 0.001 mass% or more and 3 mass% or less, and water in an amount of 80 mass% or more and 99.99 mass% or less, the treatment liquid having a pH of 5 or more and 10 or less at 25°C,

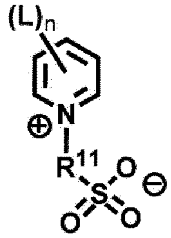

( A2-2 )

wherein $R^{11}$ is an alkylene group with 3 carbons, L is a chlorine atom, n is 1, L is bonded to a carbon atom at position 2 of the pyridine ring, and $A^-$ is $^-$OTf.

<80> A method for producing a treatment liquid for oxidation or bleaching using a liquid composition containing (A) a compound represented by the following general formula (A1-1) [hereinafter referred to as component (A)], (B) hydrogen peroxide [hereinafter referred to as component (B)] and water, the liquid composition being acidic,

wherein the treatment liquid has a pH of 5 or more and 10 or less at 25°C,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,
the method optionally including, mixing the liquid composition with water,

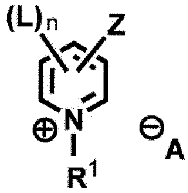

( A1-1 )

wherein $R^1$ is a methyl group, L is a chlorine atom, n is 1, L is bonded to a carbon atom at position 2 of the pyridine ring, Z is -COO-$R^2$, Z being bonded to a carbon atom at position 3 of the pyridine ring, $R^2$ is a butyl group, and $A^-$ is $^-$OTf.

<81> A method for producing a treatment liquid for oxidation or bleaching using a liquid composition containing (A) a compound represented by the following general formula (A1-1) [hereinafter referred to as component (A)], (B) hydrogen peroxide [hereinafter referred to as component (B)] and water, the liquid composition being acidic,

wherein the treatment liquid has a pH of 5 or more and 10 or less at 25°C,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,
the method optionally including, mixing the liquid composition with water,

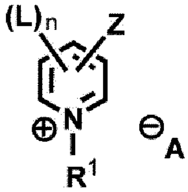

( A1-1 )

wherein $R^1$ is a methyl group, L is a chlorine atom, n is 1, L is bonded to a carbon atom at position 2 of the pyridine ring, Z is -CONH-$R^3$, Z being bonded to a carbon atom at position 3 of the pyridine ring, $R^3$ is a butyl group, and $A^-$ is $^-$OTf.

<82> A method for producing a treatment liquid for oxidation or bleaching using a liquid composition containing (A) a compound represented by the following general formula (A1-2) [hereinafter referred to as component (A)], (B) hydrogen peroxide [hereinafter referred to as component (B)] and water, the liquid composition being acidic,

wherein the treatment liquid has a pH of 5 or more and 10 or less at 25°C,
the treatment liquid contains component (A) in an amount of 0.0001 mass% or more and 1 mass% or less,
the treatment liquid contains component (B) in an amount of 0.001 mass% or more and 3 mass% or less, and

the treatment liquid contains water in an amount of 80 mass% or more and 99.99 mass% or less,
the method optionally including, mixing the liquid composition with water,

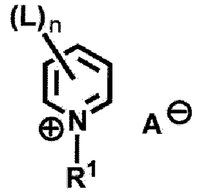

( A1-2 )

wherein $R^1$ is a dodecyl group, L is a chlorine atom, n is 1, L is bonded to a carbon atom at position 2 of the pyridine ring, and $A^-$ is $^-OTf$.

<83> A method for producing a treatment liquid for oxidation or bleaching using a liquid composition containing (A) a compound represented by the following general formula (A2-2) [hereinafter referred to as component (A)], (B) hydrogen peroxide [hereinafter referred to as component (B)] and water, the liquid composition being acidic,

wherein the treatment liquid has a pH of 5 or more and 10 or less at 25°C,
the treatment liquid contains component (A) in an amount of 0.0001 mass% or more and 1 mass% or less,
the treatment liquid contains component (B) in an amount of 0.001 mass% or more and 3 mass% or less, and
the treatment liquid contains water in an amount of 80 mass% or more and 99.99 mass% or less,
the method optionally including, mixing the liquid composition with water,

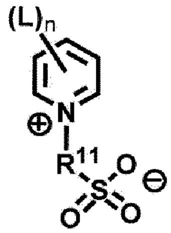

( A2-2 )

wherein $R^{11}$ is an alkylene group with 3 carbons, L is a chlorine atom, n is 1, L is bonded to a carbon atom at position 2 of the pyridine ring, and $A^-$ is $^-OTf$.

## Examples

### Synthesis example 1

3-(butoxycarbamoyl)-2-chloro-1-methylpyridin-1-ium trifluoromethanesulfonate (the compound was used in example 1-1 in Table 1-1 and others) was synthesized by the following two-step scheme.

First, an intermediate of the above compound was synthesized by the following scheme. The synthesis was performed using a 200-mL three neck eggplant flask.

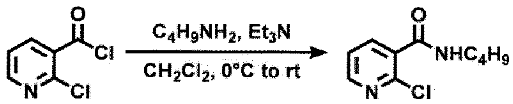


A dichloromethane (56.82 mL) solution of 2-chloronicotinic acid chloride (5 g, 28.41 mmol) was cooled to 0°C, and 2.19 g of butylamine (29.83 mmol, 1.05 eq.), and then, 4.31 g of triethylamine (42.62 mmol, 1.5 eq.), were added dropwise thereto, and stirred at room temperature (25°C) for 1 hour. After the reaction ended, a saturated ammonium chloride aqueous solution was added to the reaction solution to stop the reaction, and extraction was carried out with dichloromethane. The obtained organic layer was washed with water and a saturated sodium chloride aqueous solution, and thereafter dried with anhydrous sodium sulfate. The organic layer was concentrated with a rotary evaporator, and this was purified with column chromatography (hexane: ethyl acetate = 10:1 -> 3:1) to obtain the intermediate of the subject compound in an amount of 5.74 g (27.0 mmol, yield 95%). The $^1H$ NMR measurement results of the compound were as follows:
$^1H$ NMR (400 MHz, $CDCl_3$) $\delta$: 8.5 (1H, d, J = 4.8 Hz), 8.1 (1H, d, J = 8 Hz), 7.4 (1H, dd, J = 4.8, 8 Hz), 6.5 (1H, s), 3.5 (2H, q, J

= 5.6, 7.2 Hz), 1.7-1.7 (2H, m), 1.6-1.4 (2H, m), 1.0 (3H, t, J = 7.6 Hz) ppm.

Note that the $^1$H NMR measurement conditions were as shown below (the same applies hereinafter).

<$^1$H NMR measurement conditions>

- · Device: VNMR 400MR DD2 (manufactured by Agilent Technologies, Inc.)
- · Measurement temperature: 25°C
- · Waiting time: 10 seconds
- · Number of times of integration: 8 times · Observation range: 6410.3 Hz
- · Pulse: 45°
- · Data point: 65536

Next, a dichloromethane (34 mL) solution of the above intermediate (3.6 g, 16.9 mmol) was cooled to 0°C. Methyl trifluoromethanesulfonate (3.1 g, 18.6 mmol, 1.1 eq.) was added thereto, and stirred at room temperature (25°C) for 1 hour. After the reaction ended, the reaction end solution was concentrated with an evaporator, and the obtained residue was thereafter washed with diethyl ether to obtain the subject compound (6.1 g, yield 96%). The $^1$H NMR measurement results of the compound were as follows:
$^1$H NMR (400 MHz, $CDCl_3$) δ: = 9.0 (1H, d), 8.4 (1H, d), 8.0 (1H, s), 7.9 (1H, t), 4.4 (3H, s), 3.4 (2H, t), 1.64-1.57 (2H, m), 1.46-1.37 (2H, m), 0.9 (3H, t) ppm.

Synthesis example 2

3-(butoxycarbonyl)-2-chloro-1-methylpyridin-1-ium trifluoromethanesulfonate (the compound was used in example 1-2 in Table 1-1 and others) was synthesized by the following two-step scheme.

First, an intermediate of the above compound was synthesized by the following scheme. The synthesis was performed using a 200-mL three neck eggplant flask.

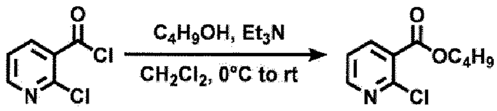


2.32 g (31.25 mmol, 1.1 eq.) of 1-butanol was added to a solution obtained from 5 g (28.41 mmol) of 2-chloronicotinic acid chloride and dichloromethane (56.82 mL), and this was cooled to 0°C. 4.32 g of triethylamine (42.62 mol, 1.5 eq.) was added dropwise thereto, and stirred at room temperature (25°C) for 1 hour. After the reaction ended, a saturated ammonium chloride aqueous solution was added to the reaction solution to stop the reaction, and extraction was carried out with dichloromethane. The obtained organic layer was washed with water and a saturated saline solution, and thereafter dried with anhydrous sodium sulfate and concentrated with a rotary evaporator to obtain a crude product. The obtained crude product was purified with silica gel column chromatography (hexane: ethyl acetate = 4:1 -> 1:1) to obtain the intermediate of the subject compound in an amount of 5.95 g (27.85 mmol, yield 98%). The $^1$H NMR measurement results of the compound were as follows:
$^1$H NMR (400 MHz, $CDCl_3$) δ: 8.5 (1H, d, J = 4.8 Hz), 8.2 (1H, d, J = 8 Hz), 7.3 (1H, dd, J = 4.8, 8 Hz), 4.4 (2H, t, J = 6.4 Hz), 1.8-1.7 (2H, m), 1.5-1.4 (2H, m), 1.0 (3H, t, J = 7.6 Hz) ppm.

Next, a dichloromethane (34 mL) solution of the above intermediate (3.6 g, 16.8 mmol) was cooled to 0°C. Methyl trifluoromethanesulfonate (3.0 g, 18.5 mmol, 1.1 eq.) was added thereto, and stirred at room temperature (25°C) for 1 hour. After the reaction ended, the reaction end solution was concentrated with an evaporator, and the obtained residue was thereafter washed with diethyl ether to obtain the subject compound (6.4 g, yield 100%). The $^1$H NMR measurement results of the compound were as follows:

$^1$H NMR (400 MHz, $CDCl_3$) δ: 9.4 (1H, d), 8.7 (1H, d), 8.1 (1H, t), 4.5 (3H, s), 4.4 (2H, t), 1.82-1.75 (2H, m), 1.5-1.4 (2H, m), 1.0 (3H, t) ppm.

Synthesis example 3

3-(2-chloropyridin-1-ium-1-yl)propane-1-sulfonate (the compound was used in example 1-3 in Table 1-1 and others) was synthesized by the following scheme. The synthesis was performed using a 200-mL one neck eggplant flask.

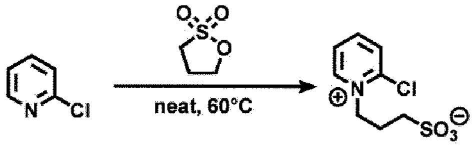


1,3-propanesultone (4.03 g, 33.02 mmol, 0.75 eq.) was added to 2-chloropyridine (5 g, 44.03 mmol), and stirred at 60°C for 5 hours. Acetone was added to the reaction end solution, the formed crystals were filtered off, and drying under reduced pressure was carried out to obtain the subject compound in an amount of 4.98 g (21.14 mmol, yield 64%). The $^1$H NMR measurement results of the compound were as follows:
$^1$H NMR (400 MHz, $D_2O$) : 9.0 (1H, d, J = 6.4 Hz), 8.5 (1H, t, J = 6.4 Hz), 8.2 (1H, d, J = 8.4 Hz), 8.0 (1H, t, J = 8.4 Hz), 5.0 (2H, t, J = 8 Hz), 3.1 (2H, t, J = 7.2 Hz), 2.5 (2H, m) ppm.

Synthesis example 4

2-chloro-1-(dodecyl)pyridin-1-ium trifluoromethanesulfonate (the compound was used in example 1-4 in Table 1-1 and others) was synthesized by the following scheme. The synthesis was performed using a 100-mL three neck eggplant flask.

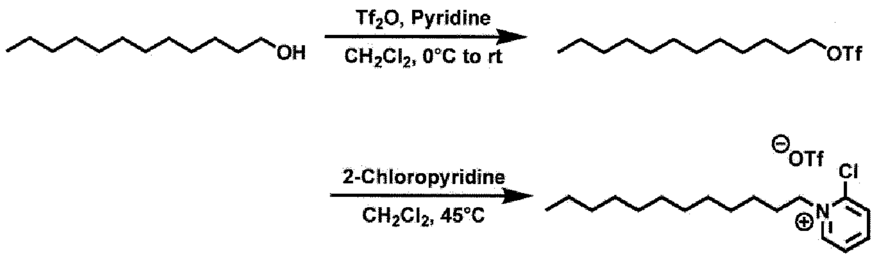


A dichloromethane (12.6 mL) solution of 1.18 g of dodecanol (6.32 mmol) was cooled to 0°C, and trifluoromethanesulfonic anhydride (2.67 g, 9.48 mmol, 1.5 eq.) and pyridine (0.55 g, 6.95 mmol, 1.1 eq.) were added dropwise thereto in succession, and stirred at 0°C for 1 hour. After the reaction ended, hexane was added to the reaction solution, the formed crystals were filtered, and the filtrate was thereafter concentrated with an evaporator. Hexane was added to the obtained residue, and the organic layer was washed with water and a saturated saline solution and thereafter dried with anhydrous sodium sulfate. After dried, the organic layer was concentrated, and the obtained residue was used for the following reaction without being purified. Dichloromethane (7.62 mL) and 2-chloropyridine (0.43 g, 3.81 mmol) were added to the residue (1.33 g, 4.19 mmol, 1.1 eq. in terms of dodecyl trifluoromethanesulfonate) obtained in the previous reaction, and stirred at 45°C. After the reaction ended, the reaction solution was concentrated with an evaporator to obtain the subject compound. The $^1$H NMR measurement results of the compound were as follows:
$^1$H NMR (400 MHz, $CDCl_3$) $\delta$: 9.1 (1H, d, J = 6 Hz), 8.5 (1H, t, J = 6.4 Hz), 8.1 (2H, m), 4.8 (2H, m), 1.9 (1H, m), 1.8 (1H, m), 1.6 (1H, m), 1.5 (1H, m), 1.4-1.2 (6H, m), 1.0 (3H, t, J = 6.8 Hz), 0.9 (6H, m) ppm.

<Reagent>

The reagents below were used in examples, comparative examples, reference examples and comparative reference examples.

- Phosphoric acid (85%): manufactured by FUJIFILM Wako Pure Chemical Corporation
- Disodium hydrogen phosphate: manufactured by FUJIFILM Wako Pure Chemical Corporation
- Potassium dihydrogen phosphate: manufactured by FUJIFILM Wako Pure Chemical Corporation
- Sodium dihydrogen phosphate: manufactured by FUJIFILM Wako Pure Chemical Corporation
- Sodium 3-(trimethylsilyl)-1-propane-1,1,2,2,3,3-d6-sulfonate: manufactured by FUJIFILM Wako Pure Chemical Corporation
- Ethanol (99.5%): manufactured by FUJIFILM Wako Pure Chemical Corporation
- Curcumin: manufactured by FUJIFILM Wako Pure Chemical Corporation
- Acetonitrile: manufactured by FUJIFILM Wako Pure Chemical Corporation
- Hydrogen peroxide (30%): manufactured by FUJIFILM Wako Pure Chemical Corporation

Those shown in parenthesis are effective amounts. The amounts in the tables are expressed in terms of effective

amounts.

[Example 1 and comparative example 1]

<Evaluation 1 of storage stability>

A phosphate buffer solution with pH = 2.5 was prepared using phosphoric acid, sodium dihydrogen phosphate and heavy water, and a solution of 26.4 mM of component (A) in Table 1-1 and 3 mM of sodium 3-(trimethylsilyl)-1-propane-1,1,2,2,3,3-d6-sulfonate (standard substance) was prepared using this. This solution corresponds to a liquid composition whose composition excluding the above standard substance is composition (mass%) in Table 1-1. The liquid compositions of the examples in Table 1-1 can be used as raw materials of oxidizing agents or bleaching agents. Immediately after prepared, the liquid composition was subjected to $^1$H NMR measurement, and stored at room temperature (25°C) after the measurement. After a lapse of 1 hour from the first measurement, $^1$H NMR measurement was carried out once again. The $^1$H NMR measurement conditions were the same as above. A reduction amount in the peaks of the evaluation compound (component (A)) was calculated from the peak ratios to the standard substance in an obtained plot, and a value of remaining rate (%) of component (A) was determined by the internal standard method to evaluate storage stability. The results are shown in Table 1-1. A higher numerical value means more excellent storage stability.

[Table 1-1]

| | | | | Example | | | |
|---|---|---|---|---|---|---|---|
| | | | | 1-1 | 1-2 | 1-3 | 1-4 |
| Liquid composition | Composition (mass%) | Component (A) | Type | 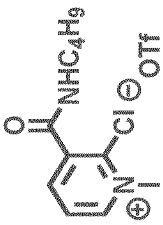 Molecular weight: 376.78 | 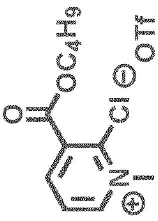 Molecular weight: 377.76 | 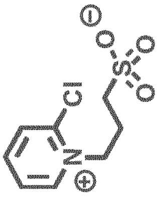 Molecular weight: 235.68 | 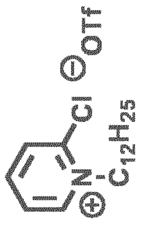 Molecular weight: 431.94 |
| | | | Mass% | 0.995 | 0.997 | 0.622 | 1.140 |
| | | Buffering agent | Phosphoric acid | 0.040 | 0.040 | 0.040 | 0.040 |
| | | | Sodium dihydrogen phosphate | 0.191 | 0.191 | 0.191 | 0.191 |
| | | Heavy water | | Balance | Balance | Balance | Balance |
| | | Total | | 100 | 100 | 100 | 100 |
| | pH (25°C) | | | 2.5 | 2.5 | 2.5 | 2.5 |
| Storage stability (evaluation 1) (%) | | | | 100 | 100 | 100 | 100 |

<Evaluation 2 of storage stability>

A carbonate buffer solution with pH = 10.5 was prepared using sodium hydrogen carbonate, sodium carbonate and heavy water, and a solution of 26.4 mM of component (A) in Table 1-2 and 3 mM of sodium 3-(trimethylsilyl)-1-propane-1,1,2,2,3,3-d6-sulfonate (standard substance) was prepared using this. This solution corresponds to a liquid composition whose composition excluding the above standard substance is composition (mass%) in Table 1-2. Storage stability was evaluated in the same manner as in the above evaluation 1 of storage stability except that this liquid composition was used. The results are shown in Table 1-2. A higher numerical value means more excellent storage stability.

[Table 1-2]

| | | | | Comparative example | | | |
|---|---|---|---|---|---|---|---|
| | | | | 1-1 | 1-2 | 1-3 | 1-4 |
| Liquid composition | Composition (mass%) | Component (A) | Type | 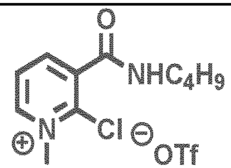 Molecular weight: 376.78 | 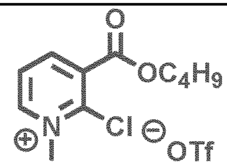 Molecular weight: 377.76 | 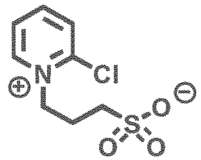 Molecular weight: 235.68 | 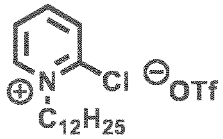 Molecular weight: 431.94 |
| | | | Mass% | 0.995 | 0.997 | 0.622 | 1.140 |
| | | Buffering agent | Sodium hydrogen carbonate | 0.109 | 0.109 | 0.109 | 0.109 |
| | | | Sodium carbonate | 0.392 | 0.392 | 0.392 | 0.392 |
| | | Heavy water | | Balance | Balance | Balance | Balance |
| | | Total | | 100 | 100 | 100 | 100 |
| | pH (25°C) | | | 10.5 | 10.5 | 10.5 | 10.5 |
| Storage stability (evaluation 2) (%) | | | | 27.4 | 7.87 | 66.67 | 13.74 |

[Example 2 and comparative example 2]

<Evaluation 3 of storage stability>

A phosphate buffer solution with pH = 2.5 was prepared using phosphoric acid, sodium dihydrogen phosphate and heavy water, and a solution of 3% of hydrogen peroxide of component (B), 26.4 mM of component (A) in Table 2 and 3 mM of sodium 3-(trimethylsilyl)-1-propane-1,1,2,2,3,3-d6-sulfonate (standard substance) was prepared using this. This solution corresponds to a liquid composition whose composition excluding the above standard substance is composition (mass%) in Table 2. The liquid compositions of the examples in Table 2 can be used as oxidizing agents or bleaching agents. Storage stability was evaluated in the same manner as in the above evaluation 1 of storage stability except that this liquid composition was used. The results are shown in Table 2. A higher numerical value means more excellent storage stability. Note that, in Table 2, compounds not qualified as component (A) are also shown in the row of component (A) for convenience.

[Table 2]

| | | | | Example | | | | Comparative example | |
|---|---|---|---|---|---|---|---|---|---|
| | | | | 2-1 | 2-2 | 2-3 | 2-4 | 2-1 | 2-2 |
| Liquid composition | Composition (mass%) | Component (A) | Type | 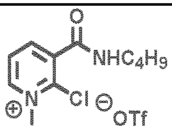 Molecular weight: 376.78 | 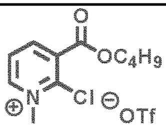 Molecular weight: 377.76 | 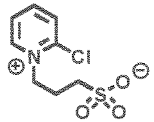 Molecular weight: 235.68 | 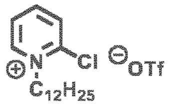 Molecular weight: 431.94 | 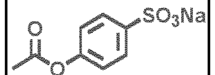 Molecular weight: 238.19 | 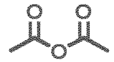 Molecular weight: 102.09 |
| | | | Mass% | 0.995 | 0.997 | 0.622 | 1.140 | 0.629 | 0.270 |
| | | Component (B) | Hydrogen peroxide | 3 | 3 | 3 | 3 | 3 | 3 |
| | | Buffering agent | Phosphoric acid | 0.040 | 0.040 | 0.040 | 0.040 | 0.040 | 0.040 |
| | | | Sodium dihydrogen phosphate | 0.191 | 0.191 | 0.191 | 0.191 | 0.191 | 0.191 |
| | | Heavy water | | Balance | Balance | Balance | Balance | Balance | Balance |
| | | Total | | 100 | 100 | 100 | 100 | 100 | 100 |
| | pH (25°C) | | | 2.5 | 2.5 | 2.5 | 2.5 | 2.5 | 2.5 |
| Storage stability (evaluation 3) (%) | | | | 95 | 95 | 100 | 94 | 100 | 0 |

[Reference example 1 and comparative reference example 1]

<Evaluation of oxidizing power>

A solution of 5.29 mM of component (A) (Table 3) (which is referred to as solution A) was prepared using ion-exchanged water, a solution of 1765 mM of component (B) (which is referred to as solution B) was prepared using ion-exchanged water and 30% hydrogen peroxide, and a solution of 1.32 mM of curcumin was prepared using ethanol. Note that, in Table 3, compounds not qualified as component (A) are also shown in the row of component (A) for convenience. 0.1 mL of solution B and 1 mL of solution A were added to 18.8 mL of a phosphate buffer solution adjusted to have pH = 7.3 using potassium dihydrogen phosphate and disodium hydrogen phosphate, and stirred for 10 seconds, and then, 0.1 mL of the curcumin solution was added thereto and stirred for 10 minutes (the final concentrations of component (A), component (B) and curcumin were 0.264 mM, 8.824 mM and 0.0066 mM, respectively). 10 minutes later, 0.5 mL of the reaction solution was taken, and 0.5 mL of a quenching liquid (0.5 M aqueous phosphate solution/acetonitrile = 1:1 (vol)) was added thereto to obtain a sample solution. This was quantified with HPLC to determine a remaining amount of curcumin. Note that this evaluation corresponds to a test for evaluating oxidative decomposition activity when the liquid compositions in Table 2 are used under a neutral condition.

As a result of the quantification with HPLC, the oxidative decomposition activity (oxidizing power (%) calculated by the formula below) of the liquid compositions in Table 3 under the neutral condition was as shown in Table 3. A higher numerical value means more excellent oxidative decomposition activity. Note that the HPLC conditions were as shown below.

Oxidizing power (%) = 100 × [(use amount of curcumin - remaining amount of curcumin)/use amount of curcumin]

<HPLC conditions>

· Device

SHIMADZU CBM-20A
Pump: LC-20A, UV-Vis detector: SPD-20A, PDA detector: SPD-M20A, column oven: CTO-20A, auto sampler: SIL-20A

· Analysis conditions

Column: L-column ODS, 150 mm × 4.6 mm, 5 μm (Chemicals Evaluation and Research Institute, Japan), oven temperature: 40°C, detector: UV (430 nm), injection volume: 10 μl, flow rate: 1.0 mL/min, mobile phase: A = 50 mM phosphoric acid, B = acetonitrile

[Table 3]

| | | | | Reference example | | | | Comparative reference example | |
|---|---|---|---|---|---|---|---|---|---|
| | | | | 1-1 | 1-2 | 1-3 | 1-4 | 1-1 | 1-2 |
| Liquid composition | Composition (mass%) | Component (A) | Type | 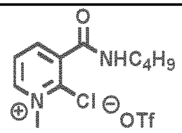 Molecular weight: 376.78 | 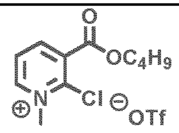 Molecular weight: 377.76 | 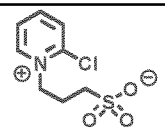 Molecular weight: 235.68 | 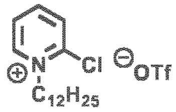 Molecular weight: 431.94 | 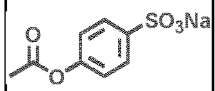 Molecular weight: 238.19 | 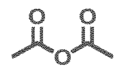 Molecular weight: 102.09 |
| | | | Mass% | 0.00995 | 0.00997 | 0.00662 | 0.0114 | 0.0062 | 0.0027 |
| | | Component (B) | Hydrogen peroxide | 0.03 | 0.03 | 0.03 | 0.03 | 0.03 | 0.03 |
| | | Buffering agent | Potassium dihydrogen phosphate | 0.1497 | 0.1497 | 0.1497 | 0.1497 | 0.1497 | 0.1497 |
| | | | Disodium hydrogen phosphate | 0.5536 | 0.5536 | 0.5536 | 0.5536 | 0.5536 | 0.5536 |
| | | Water | | Balance | Balance | Balance | Balance | Balance | Balance |
| | | Total | | 100 | 100 | 100 | 100 | 100 | 100 |
| | pH (25°C) | | | 7.3 | 7.3 | 7.3 | 7.3 | 7.3 | 7.3 |
| Oxidizing power (%) | | | | 100 | 98 | 100 | 100 | 0 | 76 |

[Reference example 2 and comparative reference example 2]

(1) Evaluation of bleaching performance

(1-1) Preparation of contaminated cloth

100 μL of grape juice (Asahi Beverages, Welch's Grape 100) or coffee (Coca-Cola (Japan) Company, Limited, GEORGIA Kaoru Blend) was added dropwise to Cotton 2003 cloth of 6 cm × 6 cm, and the cloth was dried on a vial cap overnight.

(1-2) Bleaching washing of contaminated cloth and evaluation

Bleaching washing of each contaminated cloth was performed using components (A), (B) and (C) shown in Table 4, and bleaching power was evaluated. Bleaching power evaluations were made separately on the cloth contaminated with grape juice and the cloth contaminated with coffee.

Bleaching evaluations were conducted at room temperature (25°C), and a magnetic stirrer and a stirrer were used for stirring. A phosphate buffer solution (50 mM) with pH = 7.3 was placed in a plastic cup (the amount was 80 to 100 ml per piece of contaminated cloth). In some example, a carbonate buffer solution (50 mM) with pH = 10.5 was used in place of the phosphate buffer solution to create an alkaline condition. The phosphate buffer solution is the same as in reference example 1. The carbonate buffer solution is the same as in comparative example 1 (evaluation 2 of storage stability).

A solution of 6% of hydrogen peroxide (an aqueous solution of component (B)) was added to the above cup to make an amount in terms of component (B) 0.03 mass%.

Next, an aqueous solution of component (A) was added to the above cup at a mass% in Table 4 to make an amount in terms of component (A) 0.26 mM, and 10 seconds later, each type of contaminated cloth was put thereinto. Two pieces of contaminated cloth (N = 2) were used per condition.

If a bleach activation aid as component (C) was added, after a lapse of 10 seconds from the addition of the aqueous solution of component (A), an aqueous solution of component (C) was added to the above cup at a mass% in Table 4 to make an amount in terms of component (C) 0.26 mM, and after a lapse of further 10 seconds, each contaminated cloth was put thereinto. Note that the bleach activation aid is 2-methyl-3,4-dihydroisoquinolin-2-ium tetrafluoroborate, a compound synthesized by the method described below.

After stirring at 150 to 200 rpm for 10 minutes, the contaminated cloth was taken out, and rinsed twice with stored tap water contained in a washtub.

After rinsed, the contaminated cloth was dewatered in a dewatering machine for 2 minutes, and dried naturally.

A bleaching rate was calculated by the formula below from a Z-score of a pre-washing reflectance measured in advance, a Z-score of white cloth and a post-washing Z-score. The results are shown in Table 4. The spectrophotometer SE 6000 (manufactured by NIPPON DENSHOKU INDUSTRIES CO., LTD.) was used for the reflectance measurements.

$$\text{Bleaching rate (\%)} = \frac{R_W - R_S}{R_0 - R_S} \times 100$$

$R_0$: Z-score of reflectance of white cloth
$R_S$: Z-score of pre-washing reflectance of contaminated cloth
$R_W$: Z-score of post-washing reflectance of contaminated cloth

<Synthesis example of component (C) (bleach activation aid)>

2-methyl-3,4-dihydroisoquinolin-2-ium tetrafluoroborate, the bleach activation aid of component (C), was synthesized by the scheme below. The synthesis was performed using a 50-mL one neck eggplant flask.

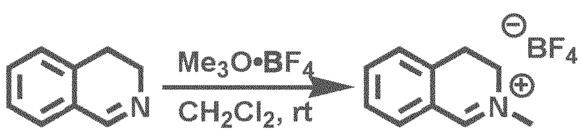


3,4-dihydroisoquinolinium (1 g, 7.6 mmol) and dichloromethane (15 ml) were added to the eggplant flask, and

stirred at room temperature (25°C). Meerwein reagent (1.24 g, 8.38 mmol, 1.1 eq.) was added dropwise thereto, and stirred at room temperature (25°C) for 1 hour. After the reaction ended, dichloromethane was distilled with an evaporator, and the obtained residue was washed with diethyl ether. After washed, the residue was dried to obtain 2-methyl-3,4-dihydroisoquinolin-2-ium tetrafluoroborate (molecular weight 233.02) (1.8 g, quant.), a target product. The $^1$H NMR measurement results of the compound were as follows (the $^1$H NMR measurement conditions are the same as above): $^1$H NMR (400 MHz, $CH_3CN$) $\delta$ = 3.24 (t, 2H), 3.70 (s, 3H), 3.97 (t, 2H), 7.45-7.56 (m, 2H), 7.74-7.79 (m, 2H), 8.74 (s, 1H).

[Table 4]

| Liquid composition | | | Reference example | | | | | Comparative reference example |
|---|---|---|---|---|---|---|---|---|
| | | | 2-1 | 2-2 | 2-3 | 2-4 | 2-5 | 2-1 |
| Composition (mass%) | Component (A) | Type | 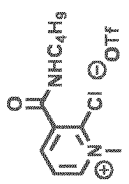 Molecular weight: 376.78 | 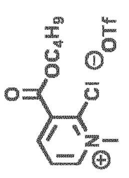 Molecular weight: 377.76 | 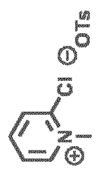 Molecular weight: 299.77 | 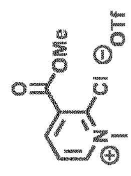 Molecular weight: 335.68 | 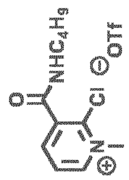 Molecular weight: 376.78 | 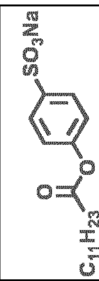 Molecular weight: 378.46 |
| | | Mass% | 0.00995 | 0.00997 | 0.00791 | 0.00886 | 0.00995 | 0.00999 |
| | Component (B) | Hydrogen peroxide | 0.03 | 0.03 | 0.03 | 0.03 | 0.03 | 0.03 |
| | Component (C) | 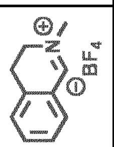 | 0.00615 | 0.00615 | 0.00615 | 0.00615 | 0.00000 | 0.00000 |
| | Buffering agent | Potassium dihydrogen phosphate | 0.1497 | 0.1497 | 0.1497 | 0.1497 | 0.1497 | 0 |
| | | Disodium hydrogen phosphate | 0.5536 | 0.5536 | 0.5536 | 0.5536 | 0.5536 | 0 |
| | | Sodium carbonate | 0 | 0 | 0 | 0 | 0 | 0.3922 |
| | | Sodium hydrogen carbonate | 0 | 0 | 0 | 0 | 0 | 0.1092 |
| | Water | | Balance | Balance | Balance | Balance | Balance | Balance |
| | Total | | 100 | 100 | 100 | 100 | 100 | 100 |
| | pH (25°C) | | 7.3 | 7.3 | 7.3 | 7.3 | 7.3 | 10.5 |
| Bleaching rate (%) | | Grape juice | 83.4 | 93.5 | 89.2 | 95 | 70.8 | 82.5 |
| | | Coffee | 70.9 | 76.6 | 76.8 | 78.1 | 56.8 | 68.5 |

[Reference example 3 and comparative reference example 3]

Bleaching power was evaluated in the same manner as in reference example 2 and others, provided that only coffee-contaminated cloth was used as contaminated cloth, the type of coffee used for preparing the contaminated cloth was changed to GEORGIA Kaoru Bito of Coca-Cola(Japan)Company, Limited, and the way in which the contaminated

cloth was dried after washed and rinsed was changed to natural drying. The results are shown in Table 5.

[Table 5]

| | | | | Reference example | | Comparative reference example | | |
|---|---|---|---|---|---|---|---|---|
| | | | | 3-1 | 3-2 | 3-1 | 3-2 | 3-3 |
| Liquid composition | Composition (mass%) | Component (A) | Type | 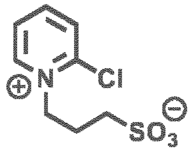 Molecular weight: 235.68 | 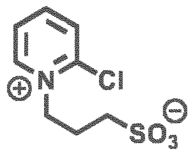 Molecular weight: 235.68 | 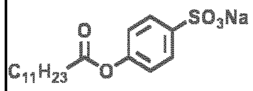 Molecular weight: 378.46 | — | — |
| | | | Mass% | 0.00622 | 0.00622 | 0.00999 | 0.00000 | 0.00000 |
| | | Component (B) | Hydrogen peroxide | 0.03 | 0.03 | 0.03 | 0.03 | 0.03 |
| | | Component (C) | 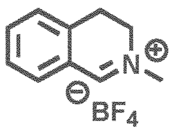 | 0.00615 | 0.00000 | 0.00000 | 0.00000 | 0.00615 |
| | | Buffering agent | Potassium dihydrogen phosphate | 0.1497 | 0.1497 | 0.1497 | 0.1497 | 0.1497 |
| | | | Disodium hydrogen phosphate | 0.5536 | 0.5536 | 0.5536 | 0.5536 | 0.5536 |
| | | Water | | Balance | Balance | Balance | Balance | Balance |
| | | Total | | 100 | 100 | 100 | 100 | 100 |
| | pH (25°C) | | | 7.3 | 7.3 | 7.3 | 7.3 | 7.3 |
| Bleaching rate (%) (coffee) | | | | 57.8 | 51.8 | 51.4 | 46.7 | 47.4 |

## Claims

1. A composition comprising, (A) one or more compounds selected from a compound represented by the following general formula (A1) and a compound represented by the following general formula (A2) [hereinafter referred to as component (A)] and water, the composition being acidic,

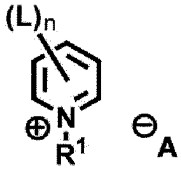

( A1 )

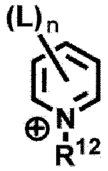

( A2 )

wherein $R^1$ is an alkyl group with 1 or more and 24 or less carbons which may contain a heteroatom, $R^{12}$ is a group with 1 or more and 24 or less carbons in total which contains a carbon atom and a heteroatom of an ion paired with $N^+$ of the pyridine ring and which may contain a heteroatom different from the heteroatom, L is n halogen atoms, n being 1, 2 or 3, and L is bonded to any n different carbon atoms at positions 2, 4 and 6 of the pyridine ring, $A^-$ is an anion, and a group other than L may be bonded to a carbon atom other than the carbon atom bonded to L of the carbon atoms of the pyridine ring.

2. The composition according to claim 1, wherein the component (A) is one or more compounds selected from a compound represented by the following general formula (A1-1), a compound represented by the following general formula (A1-2) and a compound represented by the following general formula (A2-2),

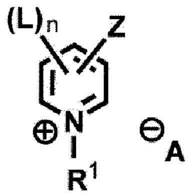

( A1-1 )

wherein in the formula (A1-1), $R^1$ is an alkyl group with 1 or more and 24 or less carbons which may contain a heteroatom, L is n halogen atoms, n being 1 or 2, and L is bonded to any n different carbons at positions 2, 4 and 6 of the pyridine ring, Z is a group selected from $-COO-R^2$, $-CONH-R^3$ and $-CON(R^4)(R^5)$, $R^2$, $R^3$, $R^4$ and $R^5$ each representing an alkyl group with 3 or more carbons which may contain a heteroatom, Z is bonded to a carbon atom different from the carbon atom bonded to L of the carbon atoms of the pyridine ring, $A^-$ is an anion, and a group other than L and Z may be bonded to a carbon atom other than the carbon atoms bonded to L and Z of the carbon atoms of the pyridine ring,

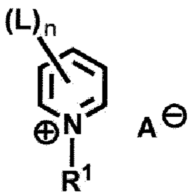

( A1-2 )

wherein in the formula (A1-2), $R^1$ is an alkyl group with 1 or more and 24 or less carbons which may contain a heteroatom, L is n halogen atoms, n being 1, 2 or 3, and L is bonded to any n different carbon atoms at positions 2, 4 and 6 of the pyridine ring, and $A^-$ is an anion, and

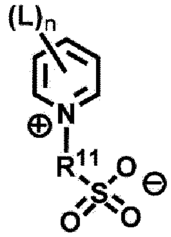

( A2-2 )

wherein in the formula (A2-2), $R^{11}$ is an alkylene group with 1 or more and 24 or less carbons which may contain a heteroatom, L is n halogen atoms, n being 1 or 2, and L is bonded to any n different carbons at positions 2, 4 and 6 of the pyridine ring, and a group other than L may be bonded to a carbon atom other than the carbon atom bonded to L of the carbon atoms of the pyridine ring.

**3.** The composition according to claim 1 or 2, further comprising (B) hydrogen peroxide [hereinafter referred to as component (B)].

**4.** The composition according to claim 3, wherein a content of the component (A) relative to 1 part by mole of the component (B) is 1 part by mole or more and 100 parts by mole or less.

**5.** The composition according to any of claims 1 to 4, wherein the composition has a pH of less than 7 at 25°C.

**6.** The composition according to any of claims 1 to 5, wherein the composition is used for oxidizing agents.

**7.** The composition according to any of claims 1 to 5, wherein the composition is used for bleaching agents.

**8.** A method for storing a compound comprising, storing (A) one or more compounds selected from a compound represented by the following general formula (A1) and a compound represented by the following general formula (A2) [hereinafter referred to as component (A)] in an acidic solution,

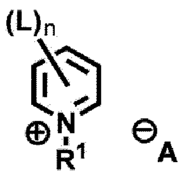

( A1 )

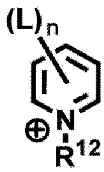

( A2 )

wherein $R^1$ is an alkyl group with 1 or more and 24 or less carbons which may contain a heteroatom, $R^{12}$ is a group with 1 or more and 24 or less carbons in total which contains a carbon atom and a heteroatom of an ion paired with $N^+$ of the pyridine ring and which may contain a heteroatom different from the heteroatom, L is n halogen atoms, n being 1, 2 or 3, and L is bonded to any n different carbon atoms at positions 2, 4 and 6 of the pyridine ring, $A^-$ is an anion, and a group other than L may be bonded to a carbon atom other than the carbon atom bonded to L of the carbon atoms of the pyridine ring.

**9.** The method for storing a compound according to claim 8, wherein the component (A) is stored under the coexistence of hydrogen peroxide.

**10.** An oxidizing method comprising, bringing a treatment liquid prepared from the composition according to any of claims 1 to 7 into contact with an object in the presence of hydrogen peroxide.

**11.** A bleaching method comprising, bringing a treatment liquid prepared from the composition according to any of claims

1 to 7 into contact with an object in the presence of hydrogen peroxide.

**12.** Use of the composition according to any of claims 1 to 5 for the production of oxidizing agents.

**13.** Use of the composition according to any of claims 1 to 5 for the production of bleaching agents.

**INTERNATIONAL SEARCH REPORT**

International application No. **PCT/JP2023/014846**

**A. CLASSIFICATION OF SUBJECT MATTER**

***C07B 33/00***(2006.01)i; ***C11D 7/18***(2006.01)i; ***C11D 7/54***(2006.01)i; ***C11D 7/60***(2006.01)i; ***C07D 213/61***(2006.01)i; ***C07D 213/80***(2006.01)i; ***C07D 213/82***(2006.01)i; ***C09K 3/00***(2006.01)i

FI: C09K3/00 109; C11D7/54; C11D7/18; C11D7/60; C07D213/80; C07D213/61; C07B33/00; C07D213/82

According to International Patent Classification (IPC) or to both national classification and IPC

**B. FIELDS SEARCHED**

Minimum documentation searched (classification system followed by classification symbols)

C07B33/00; C11D7/18; C11D7/54; C11D7/60; C07D213/61; C07D213/80; C07D213/82; C09K3/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | SANDMANN, G. et al. Quantitative structure-activity relationship of fluridone derivatives with phytoene desaturase. Pesticide Biochemistry and Physiology. (1992), vol. 42, no. 1, pp. 1-6, DOI 10.1016/0048-3575(92)90067-A<br>abstract, Materials and Methods, table 1 | 1-13 |
| A | JP 2017-538759 A (L'OREAL) 28 December 2017 (2017-12-28)<br>claims, examples, etc. | 1-13 |
| A | JP 2013-001699 A (API CORPORATION) 07 January 2013 (2013-01-07)<br>claims, examples, etc. | 1-13 |
| A | WO 2008/050732 A1 (TAKEDA PHARMACEUTICAL COMPANY LIMITED) 02 May 2008 (2008-05-02)<br>claims, examples, table 3-2, etc. | 1-13 |
| A | JP 62-166346 A (KONISHIROKU PHOTO IND CO LTD) 22 July 1987 (1987-07-22)<br>claims, examples, etc. | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 April 2023** | **27 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/014846**

| Patent document cited in search report | Publication date (day/month/year) | Patent family member(s) | Publication date (day/month/year) |
|---|---|---|---|
| JP 2017-538759 A | 28 December 2017 | WO 2016/097413 A1<br>claims, examples<br>US 2017/0340543 A1<br>CN 106999394 A | |
| JP 2013-001699 A | 07 January 2013 | (Family: none) | |
| WO 2008/050732 A1 | 02 May 2008 | US 2010/0016315 A1<br>claims, examples, table 3, etc.<br>EP 2077262 A1 | |
| JP 62-166346 A | 22 July 1987 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H3220298 A
- JP 2009155292 A
- JP 2002080894 A